# EUROPÄISCHE PATENTSCHRIFT

(11) **EP 0 336 879 B1**
(45) Veröffentlichungstag und Bekanntmachung des Hinweises auf die Patenterteilung: **22.01.1997**
(21) Anmeldenummer: 89730076.0
(22) Anmeldetag: 17.03.1989
(51) Int. Cl.: C07D 487/22, A61K 31/40

(54) **Meso-tetraphenylporphyrin-komplexverbindungen,Verfahren zu ihrer Herstellung und diese enthaltende pharmazeutische Mittel**
Meso-tetraphenylporphyrin complex compounds,process for their preparation and pharmaceuticals containing them
Complexes composés de méso-tetraphénylporphyrine,procédé pour leur préparation et agents pharmaceutiques les contenant

(30) Priorität: 18.03.1988 DE 3809671
(43) Veröffentlichungstag der Anmeldung: 11.10.1989
(73) Patentinhaber: SCHERING AKTIENGESELLSCHAFT, 13342 Berlin (DE)
(72) Erfinder: Niedballa, Ulrich, Dr., D-1000 Berlin 33 (DE); Weinmann, Hanns-Joachim, Dr., D-1000 Berlin 38 (DE); Gries, Heinz, Dr., D-1000 Berlin 31 (DE); Conrad, Jürgen, Dr., D-1000 Berlin 41 (DE); Hofmann, Sabine, D-1000 Berlin 41 (DE); Speck, Ulrich, Prof. Dr., D-1000 Berlin 28 (DE)

(56) Entgegenhaltungen:
- EP-A- 0 066 884
- EP-A- 0 071 564
- EP-A- 0 232 751
- DE-A- 3 401 052
- FR-A- 2 566 776
- PATENT ABSTRACTS OF JAPAN Band 12, Nr.373, 06.10.1988 (C-534) (3220) & JP-A- 63126880
- CANCER TREATMENT REPORTS Band 70, Nr. 3, März 1986, Seiten 391-395; N.J. Patronas et al. "Metalloporphyrin Contrast Agents for Magnetic Resonance Imaging of Human Tumors in Mice"
- JOURNAL OF HETEROCYCLIC CHEMISTRY Band 23, 1986, Seiten 1565-1570, Y. Sun et al. "The Synthesis of Two Monosubstituted meso-Tetraphenylporphine Sulfonates" Seite 1567, Schema II
- Khim. Geterotsikl. Soedin, 1987 (2), s. 221 -226
- J.Chem.Soc. Perkin Trans. I (1984), S. 1483-1487
- J.Org.Chem. Band 52, Nr.5 (1987), S.827-836
- J.Heterocycl.Chem., Band 3 (1966), S. 495-502
- J.Heterocycl.Chem., Band 23 (1986), S.561-565
- Cancer Research, Band 27, Teil 1 (1967), S.2060-2064
- Bull.Chem.Soc.Japan 1972, 45 (3), S. 664 -668
- J.Amer.Chem.Soc. 1980, 102 (22), S. 6729-6735
- Inorg.Chem. 1980, 19 (4), S. 832-836
- J.Amer.Chem.Soc., 1978, 100 (14), S. 4416-4424
- Inorg.Chem. 1978, 17 (9), S. 2666-2670
- Mag.Res.Med. 1987, 4, S. 24-33
- Sci.Pap.Inst.Phys.Chem.Res. 1976, 70 (3), S. 71-78
- J.Chem.Soc., Dalton Trans. 1981, S. 2365-2369
- Synth.React.Inorg.Met.-Org.Chem. 1983, 13 (6), S. 761-80
- J.Amer.Chem.Soc. 1977, 99 (20), S. 6594-6599
- J.Org.Chem. 1980, 45 (22), S. 4296-4302
- J.Mol.Catal. 1987, 41 (3), S. 361-366
- Inorg.Chem. 1982, 21 (12), S. 4248-4253 J.Amer.Chem.Soc. 1984, 106 (23), S. 6888-6898
- J. Amer.Chem.Soc.1984, 106 (23), S. 6888-6898

## Beschreibung

Die Erfindung betrifft den in den Patentansprüchen gekennzeichneten Gegenstand, das heißt paramagnetische meso-Tetraphenylporphyrin-Komplexverbindungen, diese Verbindungen enthaltende neue pharmazeutische Mittel, ihre Verwendung in der Diagnostik sowie Verfahren zur Herstellung dieser Verbindungen und Mittel.

Die Anwendung von Komplexbildnern oder Komplexen bzw. deren Salzen in der Medizin ist seit langem bekannt. Als Beispiele seien genannt:

Komplexbildner als Stabilisatoren pharmazeutischer Präparate, Komplexe und deren Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen, als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotope wie ^{99m}Tc für die Szintigraphie sind bekannt.

In den Patentschriften EP71564, EP 130934 und DE-OS 3401052 sind neuerdings Komplexe und Komplexsalze als Diagnostika, vorwiegend als NMR-Diagnostika, vorgestellt worden.

Diese Komplexe und Komplexsalze sind recht gut verträglich und gewährleisten eine weitestgehende vollständige Ausscheidung der Ionen. Sie erfüllen jedoch noch nicht optimal alle die relative Wirksamkeit eines NMR-Kontrastmittels bestimmenden Kriterien, von denen vor allem zu nennen sind:
eine starke NMR-Aktivität (Relaxivität), so daß das Kontrastmittel in möglichst geringen Konzentrationen die Relaxationszeiten der Protonen im Gewebewasser und anderer Kerne wie P, F und Na in vivo erniedrigt und damit die Lokalisation von Tumoren durch Erhöhung der Signalintensität des mit Hilfe des Kernspintomographen erhaltenen Bildes ermöglicht; eine möglichst selektive Anreicherung und/oder Retention des Kontrastmittels am Zielorgan bzw. cancerösem Gewebe; ausreichende Wasserlöslichkeit; geringe Toxizität; gute Verträglichkeit; gute chemische und biochemische Stabilität.

Für die Bildgebung relevant sind dabei vor allem die beiden erstgenannten Punkte. Da die Relaxationszeiten zwischen den Geweben sich meistens nur um den Faktor 2-3 unterscheiden (T.E. Budinger und P.C. Lauterbur, Science 226, pp 288-298, 1984; J.M.S. Hutchinson und F.W. Smith in Nuclear Magnetic Resonance Imaging Edit. C.L. Partain et al., pp 231-249, Saunders, New York 1983) und die Komplexe und Komplexsalze der genannten Patentschriften im allgemeinen mit dem Nachteil behaftet sind, daß sie sich nur relativ unspezifisch im Extrazellulärraum verteilen und daher nicht immer für eine Erkennung pathologisch veränderter Gewebe taugen, besteht ein Bedarf nach vor allem selektiv-bindenden, tumorspezifischen Verbindungen, die sich in der Diagnostik verwenden lassen.

Es ist nun seit einigen Jahren bekannt, daß sich Porphyrinderivate selektiv in menschlichen und tierischen Tumoren anreichern (D. Kessel und T.-H. Chou, Cancer Res. 43, pp 1994-1999, 1983, P. Hambright, Bioinorg. Chem. 5, pp 87-92, 1975; R. Lipson et al., Cancer 20, pp. 2250-2257, 1967; D. Sanderson et al.,, Cancer 30, pp. 1368-1372, 1972). Erste Ansätze, diese Verbindungsklasse auch als Diagnostika einzusetzen sind ebenfalls beschrieben worden (J. Winkelmann et al., Cancer Research 27, pp. 2060-2064, 1967; Europäische Patentanmeldung Publikations Nr. 133603; N. J. Patronas et al., Cancer Treatment Reports 70, pp. 391-395, 1986). Bei diesen Verbindungen (Tetrakis(4-sulfonatophenyl)- und Tetrakis(4-carboxylatophenyl)-porphyrin) hat sich als paramagnetisches Metall nur das Mangan(III)-Ion als geeignet erwiesen.

Die bisher beschriebenen Verbindungen sind jedoch weit davon entfernt die oben genannten Kriterien zufriedenstellend zu erfüllen; besondere Aufmerksamkeit verlangt immer noch ihre mangelnde Anreicherung in den Zielorganen. Eine Verbesserung dieser Eigenschaft sollte gleichzeitig die bestehenden Probleme mit der Toxizität und Verträglichkeit der vorbekannten Verbindungen reduzieren helfen.

Es besteht daher weiterhin für vielfältige Zwecke ein Bedarf an stabilen, gut löslichen, aber auch besser verträglichen, selektiver bindenden, gut zugänglichen, über eine größere chemische Variationsbreite der Substituenten (die z.B. den Einbau anderer Metalle als Mangan bzw. mehrerer, auch unterschiedlicher, Metalle ermöglicht und damit gleichzeitig auch zu einer Steuerung der Eigenschaften und Anwendungen der Verbindungen führt) verfügenden Komplexverbindungen, die für die Diagnostik von Tumoren geeignet sind.

Der Erfindung liegt somit die Aufgabe zugrunde, diese Verbindungen und pharmazeutischen Mittel zur Verfügung zu stellen, sowie ein Verfahren zu ihrer Herstellung zu schaffen.
Diese Aufgabe wird durch die Erfindung gelöst.

Es wurde gefunden, daß sich Komplexverbindungen, bestehend aus einem meso-Tetraphenylporphyrinliganden, mindestens einem paramagnetischen Ion eines Elements der Ordnungszahlen 13, 21-32, 37-39, 42-44, 49, 50 oder 57-83 sowie gegebenenfalls Kationen anorganischer und/oder organischer Basen, Aminosäuren oder Aminosäureamide, überraschenderweise hervorragend zur Herstellung von NMR-Diagnostika eignen.

Die erfindungsgemäßen pharmazeutischen Mittel weisen als Porphyrinliganden Verbindungen der allgemeinen Formel I auf 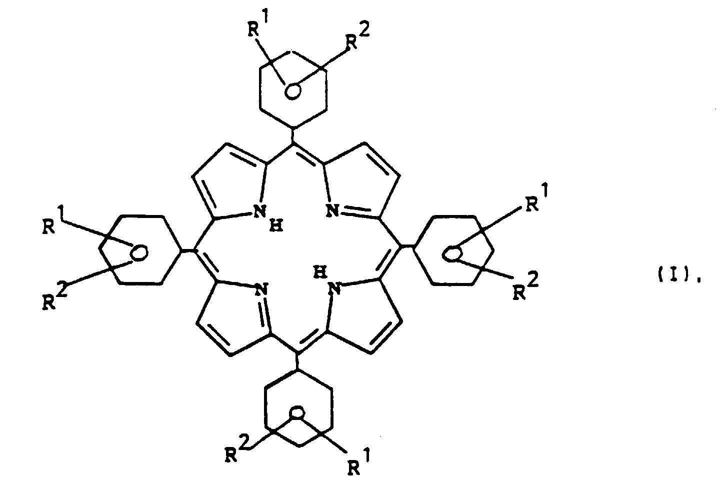 worin
R¹ für einen CO-A-, W- oder NH-W-Rest mit
   A in der Bedeutung einer (NH)ₓ-[Q-(NH)_{y}]_{w}-W-Gruppe.
x und y die Ziffern 0, 1 oder 2
w die Ziffern 0 oder 1,
Q eine C₁-C₂₀-Alkylengruppe.
W die Gruppierung V-K bedeutet, wobei
   V eine gegebenenfalls Imino-, Polyethylenoxy-, Phenylen-, Phenylenoxy-, Phenylenimino-. Amido-, Hydrazido-, Estergruppe(n), Sauerstoff-, Schwefel- und/oder Stickstoff-Atom(e) enthaltende gegebenenfalls durch Hydroxy-, Mercapto-, Imino-, Epoxy-, Oxo-, Thioxo- und/oder Aminogruppe(n) substituierte geradkettige, verzweigte, gesättigte oder ungesättigte C₁-C₂₀-Alkylengruppe oder eine direkte Bindung,
   K ein Komplexbildner der allgemeinen Formeln IA, I B oder I C 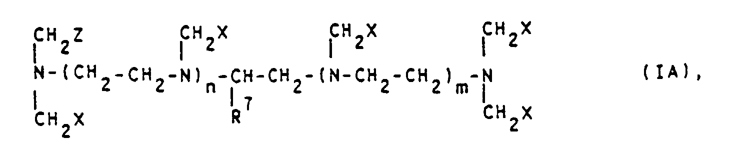 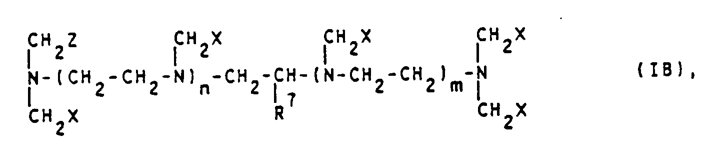 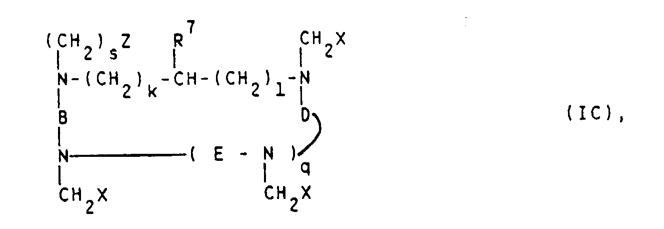
bedeuten,
wobei
n und m jeweils für die Ziffern 0, 1, 2, 3 oder 4, wobei n und m
zusammen nicht mehr als 4 ergeben,
k für die Ziffern 1, 2, 3, 4 oder 5,
1 für die Ziffern 0, 1, 2, 3, 4 oder 5,
q für die Ziffern 0, 1 oder 2,
s für die Ziffern 0 oder 1,
X für -COOH.
B, D und E, die gleich oder verschieden sind, jeweils für die Gruppe 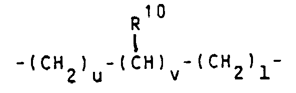 mit
   R¹⁰ in der Bedeutung von Wasserstoff oder einer gegebenenfalls Sauerstoff-und/oder Stickstoffatom(e) enthaltenden gegebenenfalls durch Hydroxy-und/oder Aminogruppe(n) substituierten geradkettigen, verzweigten, gesättigten oder ungesättigten C₁-C₂₀-Alkylgruppe,
   u in der Bedeutung der Ziffern 0, 1, 2, 3, 4 oder 5,
   v in der Bedeutung der Ziffern 0 oder 1,
   wobei B, D und E jeweils mindestens 2 und maximal 5 Kohlenstoffatome enthalten,
Z für die Gruppe 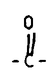 oder den Rest X,
R⁷ für eine direkte Bindung oder ein Wasserstoffatom, stehen, mit der Maßgabe, daß Z nur dann für die Gruppe 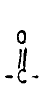 steht, wenn R⁷ gleichzeitig ein Wasserstoffatom bedeutet, und daß Z nur dann für den Rest X steht, wenn R⁷ gleichzeitig eine direkte Bindung und s die Ziffer 1 bedeuten,
R² für ein Wasserstoff-, Fluor-, Chlor-, Brom-, Jod-atom oder R⁴, wobei R⁴ einen C₁-C₆-Alkyl- oder Benzylrestbedeutet, steht, wobei gewünschtenfalls noch vorhandene freie Carbonsäuregruppen teilweise oder vollständig als Ester und/oder Amid oder als Salz einer anorganischen und/oder organischen Base, Aminosäure oder Aminosäureamid vorliegen mit der Maßgabe, daß das gegebenenfalls im Porphyrin-Gerüst enthaltene Metall Mangan ist.

Die Erfindung betrifft weiterhin Komplexverbindungen der allgemeinen Formel I, worin die Substituenten die oben genannten Bedeutungen haben und gegebenenfalls mindestens einem Ion eines Elements der Ordnungszahlen 13, 21-32, 37-39, 42-44, 49, 50 oder 57-83 sowie gegebenenfalls Kationen anorganischer und/oder organischer Basen, Aminosäuren oder Aminosäureamide.

Die in J. Heterocyclic Chem. 3,495 (1966) beschriebene Synthese der zuletzt genannten metallfreien Porphyrine wurde für geplante Untersuchungen in verschiedenen biochemischen und biologischen Systemen (z.B. Bindung an Proteine und Nucleinsäuren, Gewebeverteilung) durchgeführt.

Die erfindungsgemäßen Komplexverbindungen umfassen insgesamt zwei Gruppen von Verbindungen: a) Verbindungen, die Metallionen im Komplexbildner-Rest K enthalten; und b) Verbindungen, die sowohl Metallionen im Porphyrinliganden als auch im Komplexbildner-Rest K gebunden enthalten, wobei die Metallionen unterschiedlich sein können.

Da die erfindungsgemäßen Mittel zur Anwendung in der NMR-Diagnostik bestimmt sind, müssen paramagnetische Metallionen im Komplex vorhanden sein. Dies sind insbesondere die zwei- und dreiwertigen Ionen der Elemente der Ordnungszahlen 21-29, 42, 44 und 57-70. Geeignete Ionen sind beispielsweise das Chrom(III)-, Mangan(II)-, Mangan(III)-, Eisen(II)-, Cobalt(II)-, Nickel(II)-, Kupfer(II)-, Praseodym(III), Neadym(III)-, Samarium(III)- und Ytterbium(III)-ion. Wegen ihres hohen magnetischen Moments sind besonders bevorzugt das Gadolinium(III), Terbium(III)-, Dysprosium(III)-, Holmium(III)-, Erbium(III)- und Eisen(III)ion.

Ein wesentlicher Vorteil der erfindungsgemäßen, den Komplexbildner-Rest K enthaltenden Metallkomplexe ist, daß bei diesen der vom Metallion bewirkte diagnostische Effekt durch den Einbau eines weiteren Metallions verstärkt werden kann, bzw. durch den Einbau eines anderen, vom ersten unterschiedlichen Metallions, insbesondere die physikalischen Eigenschaften durch eine Verstärkung der magnetischen Effekte der Komplexverbindungen verbessert werden können.

Überraschend ist hierbei, daß die wesentlichen, die Wirksamkeit dieser pharmazeutischen Mittel bestimmenden Eigenschaften, wie zum Beispiel vor allem die hohe Selektivität und Anreicherung der Komplexe, erhalten bleiben oder verbessert werden.

Mit Hilfe der erfindungsgemäßen Komplexverbindungen können überraschenderweise nicht nur Tumorgewebe und einzelne Organe, wie zum Beispiel Leber und Nieren, sondern auch Blutgefäße ohne Anwendung spezieller Puls-Sequenzen in-vivo dargestellt werden, womit sie unter anderem als Perfusionsagentien Verwendung finden können.

Als Alkylsubstituent R⁴ kommen Kohlenwasserstoffe mit 1-6, vorzugsweise 1-4 C-Atomen, wie zum Beispiel Methyl-, Ethyl-, Propyl-, Isopropyl-, n-, sek.- oder tert.-Butyl-, Isobutyl-, Pentyl- und Hexylreste in Betracht.

Die für V stehende Alkylengruppe bzw. die für R¹⁰ stehende Alkylgruppe kann geradkettig, verzweigt, cyclisch, aliphatisch, aromatisch oder arylaliphatisch sein, bis zu 20 Kohlenstoffatome aufweisen und gegebenenfalls -NH-, -O-, -S-, -N-, -CO-O-, -O-CO-, (0-CH₂CH₂-)poly, -NH-CO-, -CO-NH-, -NH-NH-, -C₆H₄-NH-, -C₆H₄-O-, -C₆H₄- enthalten. Bevorzugt sind die geradkettigen Mono- bis Decamethylengruppen sowie C₁-C₄-Alkylenphenylgruppen.
Zur Verdeutlichung seien folgende Alkylengruppen beispielhaft genannt:
-(CH₂)₂NH-; -CH₂-O-C₆H₄-CH₂-; -CH₂-CH(OH)-CH₂-O-C₆H₄-CH₂-; -C(=NH)-O-C₆H₄-CH₂; (CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O- C₆H₄-CH₂-; -CH₂-CO-NH-(CH₂)₃-O-CH₂-; -CH₂-CO-NH-NH-; -CH₂-CO-NH(CH₂)₂-; CH₂-CO- -NH(CH₂)₁₀-;-CH₂-CO-NH-(CH₂)₂-S-; -(CH₂)₄-NH-CO-(CH₂)₈-; -CH₂-CO-NH-(CH₂)₃-NH-; -(CH₂)₃-NH-; -(CH₂)-NH-C(=S)-NH-C₆H₄-CH₂-; -(CH₂)₂-NH-CO-CH₂-(OCH₂CH₂)₄₃-OCH₂-.

Als Beispiele für die Komplexbildner-Reste K seien diejenigen der Ethylendiamintetraessigsäure, Diethylentriaminpentaessigsäure, 1,4,7,10-Tetraazacyclododecantetraessigsäure, 1,4,7-Triazacyclononantriessigsäure, 1,4,8,11-Tetraazatetradecantetraessigsäure und 1,5,9-Triazacyclododecantriessigsäure genannt, die über (jeweils in K enthaltend) ein Kohlenstoffatom oder eine Carbonylgruppe an die jeweiligen Porphyrinderivate gebunden sind. Gewünschtenfalls kann ein Teil der Carbonsäuren als Ester und/oder Amid vorliegen.

Die restlichen aciden Wasserstoffatome, das heißt diejenigen, die nicht durch das Zentralion substituiert worden sind, können gegebenenfalls ganz oder teilweise durch Kationen anorganischer und/oder organischer Basen oder Aminosäuren ersetzt sein. Die entsprechenden Säuregruppen können auch ganz oder teilweise in Ester oder Amide überführt sein.

Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.
Geeignete Ester sind vorzugsweise diejenigen mit einem der für R⁴ genannten Reste; aufgeführt seien beispielsweise der Methyl-, Ethyl- und tertiär-Butylrest.
Sollen die Carbonsäuregruppen zumindest teilweise als Amide vorliegen, so sind bevorzugt tertiäre Amide mit gesättigten, ungesättigten, gerad- oder verzweigtkettigen oder cyclischen Kohlenwasserstoffresten mit bis zu 5 C-Atomen, die gegebenenfalls durch 1 bis 3 Hydroxy- oder C₁-C₄-Alkoxy-Gruppen substituiert sind. Beispielsweise genannt seien die Methyl-, Ethyl-, 2-Hydroxyethyl-, 2-Hydroxy-1-(hydroxymethyl)-ethyl-, 1-(Hydroxymethyl)-ethyl-, Propyl-, Isopropenyl, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-,2-, 3- und 4-Hydroxy2-methylbutyl-, 2- und 3-Hydroxyisobutyl-, 2,3,4-Trihydroxybutyl-, 1,2,4-Trihydroxybutyl-, Pentyl-, Cyclopentyl- und 2-Methoxyethyl-gruppe.
Als bevorzugte heterocyclische Amidreste seien der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-Ring genannt.
Die Herstellung der erfindungsgemäßen meso-Tetraphenylporphyrin-Komplexverbindungen erfolgt dadurch, daß man in Porphyrinen der allgemeinen Formel II 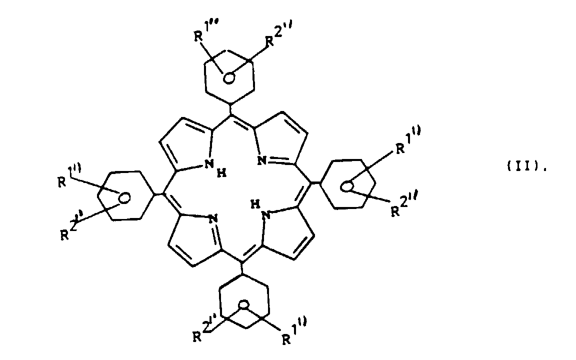 worin
R^{1"} für R^{1'} oder einen Substituenten, der in R^{1'} umgewandelt werden kann,
R^{2"} für ein Wasserstoff-, Fluor-, Chlor-, Brom-, Jod-atom- oder, R⁴ stehen,
   wobei
R^{1'} und R^{2'} die für R¹ und R² angegebene Bedeutung haben, der Substituent W jedoch für ein Wasserstoffatom steht,
gegebenenfalls in an sich bekannter Weise in Porphyrine der allgemeinen Formel II' überführt, worin R^{1"} für R^{1'}, R^{2"} für R^{2'} stehen und anschließend
a) die pyrrolischen NH's durch das Manganatom substituiert, gegebenenfalls vorhandene Schutzgruppen entfernt und anschließend den Rest V-K einführt und danach mit mindestens einem Metalloxid oder Metallsalz eines Elements der Ordnungszahlen 21-29, 42, 44 oder 57-70 umsetzt oder
b) den Substituenten V-K einführt, mit mindestens einem Metalloxid oder Metallsalz eines Elements der Ordnungszahlen 21-29, 42, 44 oder 57-70 umsetzt und anschließend gewünschtenfalls die pyrrolischen NH's durch ein Manganatom substituiert oder
c) den Substituenten V-K einführt, die pyrrolischen NH's durch ein Manganatom substituiert und anschließend mit mindestens einem Metalloxid oder Metallsalz eines Elements der Ordnungszahlen 21-29, 42, 44 oder 57-70 umsetzt
und gegebenenfalls anschließend in den nach a), b) oder c) erhaltenen Komplexverbindungen noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester und/oder Amide überführt.
Man geht dabei aus von käuflich zu erwerbenden [z.B. meso-Tetra-(4-methoxyphenyl)-porphyrin bei Aldrich Chemie GmbH, Steinheim; siehe Beispiel 17] oder von literaturbekannten bzw. analog literaturbekannten Methoden herstellbaren Porphyrin-Edukten [z.B. meso-Tetra-(4-sulfonatophenyl)porphyrin: J. Winkelman et al., Cancer Res. 27, 2060, 1967; meso-Tetra-(4-aminophenyl)-porphyrin: A.S. Semeikin et al., C.A. 105, 1986, 133611h; meso-Tetra-(4-carboxyphenyl)-porphyrin: J. Lindsey et al., J. Org. Chem. 52, 827, 1987; meso-Tetra-(3-trifluormethylphenyl)-porphyrin: ringsynthetisch analog Y. San et al., J. Heterocyclic Chem. 23, 561, 1986, Falk, Porphyrins and Metalloporphyrins, Elsevier 1975, D. Dolphin, The Porphyrins, Academic Press, 1978; K.M. Smith, Porphyrins and Metalloporphyrins, Elsevier, Amsterdamm, 1975).
Sie werden in an sich bekannter Weise in die Porphyrinderivate der allgemeinen Formel II' umgewandelt, z.B. durch Sulfonierung (R.D. Macfarlane et al., J. Heterocyclic Chem. 23, 1565, 1986; Houben-Weyl, Methoden der Organischen Chemie, Band IX, 1955, S. 450, Georg Thieme Verlag Stuttgart). Hydrolyse von CF₃-Substituenten zu Carboxylgruppen (Houben-Weyl, Methoden der Organischen Chemie, Band V/3, 1962, S. 473, Georg Thieme Verlag, Stuttgart), Nitrierung und anschließende Reduktion zur Aminogruppe (Y. Sun et al., J. Heterocyclic Chem., 23, 561, 1986, Houben-Weyl, Methoden der Organischen Chemie, Band X/1, 1971, S. 478, Georg Thieme Verlag, Stuttgart), Veresterung (L.R. Milgram, J. Chem. Soc. Perkin Trans I 1984, 1483) usw.

Zur Herstellung von aminoalkylensubstituierten Porphyrin-Amidderivaten (die als Edukte für den Substituenten K enthaltende Porphyrinkomplexe dienen können) werden aktivierte Porphyrin-Säurederivate mit endständigen Alkylendiaminen umgesetzt, von denen eine Aminogruppe zum Beispiel in Form des Carbobenzoxyrestes geschützt ist. Die Entfernung der Schutzgruppe erfolgt anschließend nach literaturbekannten Methoden, zum Beispiel durch Behandlung mit Trifluoressigsäure.

Zur Einführung des Komplexbildner-Restes K werden die so erhaltenen Verbindungen in an sich bekannter Weise mit isothiocyanatobenzyl-substituierten Komplexbildnern umgesetzt (0. Gansow et al., Inorg. Chem. 25, 2772, 1986), amidiert, hydraziniert (Houben-Weyl. Methoden der organischen Chemie, Band VIII/3 Georg Thieme Verlag, Stuttgart (1952), 654 und 676), acyliert (J. March, Advanced Organic Chemistry, McGraw-Hill, 2nd ed.,(1977) 377-382) und/oder alkyliert (Houben-Weyl, Methoden der organischen Chemie, Band VI/3 Georg Thieme Verlag, Stuttgart (1965), 187).
Als Substrat zur Einführung der Einheiten V-K dienen Verbindungen der allgemeinen Formeln I'A, I'B, I'C, I"AB, I"C 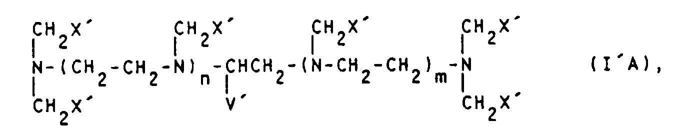 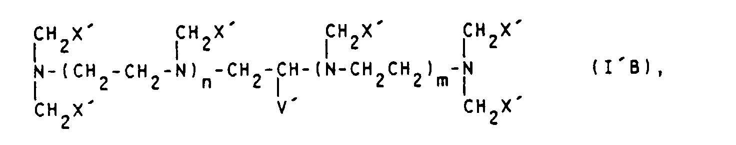 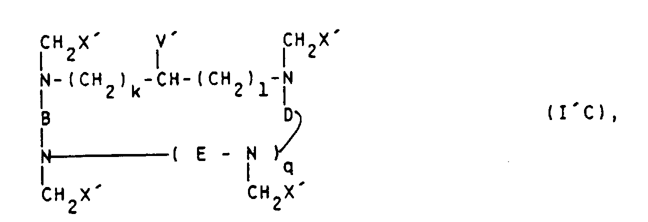 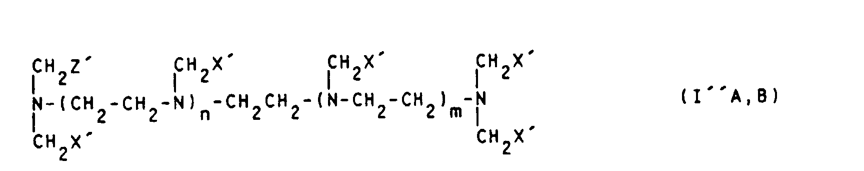 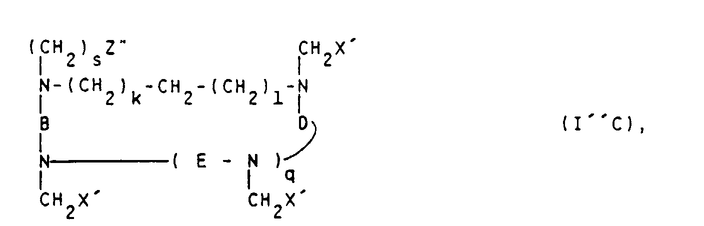 worin V' für einen in V umzuwandelnden Substituenten, X' für COOY mit Y in der Bedeutung von Wasserstoff oder einer Säureschutzgruppe, Z' für eine aktivierte Carbonylgruppe und Z" für Z' oder im Falle von s = 0 für Wasserstoff stehen.

Im letztgenannten Fall (I"C mit s=0 und Z"=H) werden die Verbindungen der allgemeinen Formel I"C mit aktivierten Porphyrinsäurederivaten umgesetzt.

Als Beispiel für eine aktivierte Carbonylgruppe seien Anhydrid (dieses kann auch mit der benachbarten Säuregruppe des gleichen Moleküls gebildet sein), p-Nitrophenylsäureester und Säurechlorid genannt.

Als Säureschutzgruppen Y kommen niedere Alkyl-, Aryl- und Aralkylgruppen, beispielsweise die Methyl-, Ethyl-, Propyl-, Butyl-, Phenyl-, Benzyl-, Diphenylmethyl, Triphenylmethyl-, bis-(p-Nitrophenyl)-methylgruppe, sowie Trialkylsilylgruppen in Frage.

Die Abspaltung der Schutzgruppen Y erfolgt nach den dem Fachmann bekannten Verfahren, beispielsweise durch Hydrolyse, alkalische Verseifung der Ester mit Alkali in wäßrig-alkoholischer Lösung bei Temperaturen von 0°C bis 50°C oder im Fall von tert.-Butylestern mit Hilfe von Trifluoressigsäure.

Die zur Einführung der Komplexbildner-Einheiten vorgenommene Alkylierung bzw. Acylierung wird mit Reagenzien durchgeführt, die den gewünschten K-V- Substituenten (gebunden an eine Fluchtgruppe) enthalten oder aus denen der gewünschte Substituent, gegebenenfalls nach Modifikation durch Folgereaktion(en), durch die Reaktion generiert wird. Als Beispiele für die erstgenannten seien Halogenide, Mesylate, Tosylate und Anhydride genannt. Zur zweiten Gruppe gehören zum Beispiel Oxirane, Thiirane, Azirane, α,β-ungesättigte Carbonylverbindungen oder deren Vinyloge, Aldehyde, Ketone, Isothiocyanate und Isocyanate.

Als Beispiele für Folgereaktionen seien Hydrierungen, Veresterungen, Oxidationen, Veretherungen und Alkylierungen genannt, die nach dem Fachmann bekannten Literaturverfahren durchgeführt werden.

Die als Edukte benötigten Verbindungen I' sind bekannt (Z.B. Europäische Patentanmeldung Publikation Nr. 0154788) oder können aus den entsprechenden Polyaminen (wobei vorhandene funktionelle Gruppen gegebenenfalls geschützt sind) durch Alkylierung mit einem Ester der allgemeinen Formel III

HalCH₂COOY (III),

worin Hal für Chlor, Brom oder Jod steht, hergestellt werden.

Die Umsetzung erfolgt in polaren aprotischen Lösungsmitteln wie zum Beispiel Dimethylformamid, Dimethylsulfoxid oder Hexamethylphosphorsäuretriamid in Gegenwart eines Säurefängers, wie zum Beispiel tertiäres Amin (zum Beispiel Triäthylamin, Trimethylamin, N,N-Dimethylaminopyridin, 1,5-Diazabicyclo[4.3.0], nonen-5(DBN), 1,5-Diazabicyclo[5.4.0]-undecen-5-(DBU), Alkali-, Erdalkalicarbonat oder -hydrogencarbonat (zum Beispiel Natrium-, Magnesium-, Calcium-, Barium-, Kalium- carbonat und -hydrogen-carbonat) bei Temperaturen zwischen -10°C und 120°C, vorzugsweise zwischen 0°C und 50°C.

Die Herstellung der aktivierten Carbonylderivate I" (z.B. gemischtes Anhydrid, N-Hydroxysuccinimidester, Acylimidazole, Trimethylsilylester) erfolgt nach literaturbekannten Methoden [Houben-Weyl, Methoden der organischen Chemie, Georg Thieme Verlag, Stuttgart, Band E 5(1985), 633; Org. React. 12,157(1962)] oder wird im experimentellen Teil beschrieben.

Die für die Herstellung der Polyamin-polysäuren der allgemeinen Formel I'A als Edukte benötigten entsprechenden Polyamine werden analog literaturbekannten Methoden (zum Beispiel Canad. Patent No. 1 178 951, Eur. I. Med. Chem.-Chim.Ther. 1985,20,509 und 1986, 21,333) hergestellt, indem man von Aminosäuren ausgeht, die in gegebenenfalls ethylenaminsubstituierte Amide (zum Beispiel mit N-(2-Aminoethyl)-carbaminsäurebenzylester) überführt und anschließend (gegebenenfalls nach Abspalten der Schutzgruppen) zu den gewünschten Aminen (vorzugsweise mit Diboran oder Lithiumaluminiumhydrid) reduziert werden.

Will man die Polyamin-Edukte für die Verbindungen der allgemeinen Formel I'B synthetisieren, so ist es notwendig, vor der Reduktion ein derartiges Amid durch Umsetzung mit zum Beispiel Ethyloxamat in einem polaren Lösungsmittel wie zum Beispiel Tetrahydrofuran, Dimethylsulfoxid oder Dimethoxyethan bei einer Temperatur zwischen 50°C und 250°C, vorzugsweise 70°C bis 150°C (gegebenenfalls in einem Druckbehälter) an der α-Aminogruppe zu substituieren, so daß man ein 3-Aza-2-oxo-glutarsäurediamid-Derivat als Zwischenprodukt erhält.

Die Herstellung der als Edukte für I'C bzw. I"C benötigten cyclischen Polyamine erfolgt durch Cyclisierung zweier Reaktanten, von denen (im Falle der Synthese von I'C) der eine V'-substituiert ist.

Die Cyclisierung wird nach literaturbekannten Methoden, zum Beispiel Org. Synth. 58,86 (1978), Macrocyclic Polyether Syntheses, Springer Verlag, Berlin, Heidelberg, New York 1982, Coord. Chem. Rev. 3,3 (1968), Ann. Chem. 1976, 916, durchgeführt: einer der beiden Reaktanten trägt am Kettenende zwei Fluchtgruppen, der andere zwei Stickstoffatome, die nukleophil diese Fluchtgruppen verdrängen. Als Beispiel seien genannt die Umsetzung von endständigen, gegebenenfalls ein oder zwei Stickstoffatom(e) enthaltenden, Dibrom-, Dimesyloxy-, Ditosyloxy- oder Dialkoxycarbonylalkylenverbindungen mit endständigen, gegebenenfalls ein oder zwei zusätzliche Stickstoffatom(e) in der Alkylenkette enthaltenden Diazaalkylenverbindungen, von denen (im Falle der Synthese von I'C) einer der beiden Reaktanten V'-substituiert ist.

Die Stickstoffatome sind gegebenenfalls geschützt, zum Beispiel als Tosylate, und werden vor der nachfolgenden Alkylierungsreaktion nach literaturbekannten Verfahren freigesetzt.

Werden Diester in die Cyclisierungsreaktion eingesetzt, so müssen die so erhaltenen Diketoverbindungen nach dem Fachmann bekannten Verfahren, zum Beispiel mit Diboran, reduziert werden.
Als Substituenten V', der in V überführt werden kann, sind unter anderem Hydroxy-und Nitrobenzyl-, Hydroxy-und Carboxyalkyl- sowie Thioalkylreste mit bis zu 20 Kohlenstoffatomen geeignet. Sie werden nach dem Fachmann bekannten Literaturverfahren (Chem. Pharm. Bull. 33,674 (1985), Compendium of Org. Synthesis Vol. 1-5, Wiley and Sons, Inc., Houben-Weyl, Methoden der organischen Chemie, Band VIII, Georg Thieme Verlag, Stuttgart, J. Biochem. 92, 1413,1982) in die gewünschten Substituenten ( zum Beispiel mit der Amino-, Hydrazino-, Hydrazinocarbonyl-, Epoxid-, Anhydrid-, Halogeno-, Halogenocarbonyl-, Mercapto-, Isothiocyanatgruppe als funktioneller Gruppe) umgewandelt, wobei im Falle des Nitrobenzylrestes zunächst eine katalytische Hydrierung (zum Beispiel nach P.N. Rylander, Catalytic Hydrogenation over Platinum Metals, Academic Press 1967) zum Aminobenzylderivat vorgenommen werden muß.
Beispiele für die Umwandlung von an aromatische oder aliphatische Reste gebundenen Hydroxy- oder Aminogruppen sind die in wasserfreien, aprotischen Lösungsmitteln wie Tetrahydrofuran, Dimethoxyethan oder Dimethylsulfoxid in Gegenwart eines Säurefängers wie zum Beispiel Natriumhydroxid, Natriumhydrid oder Alkali- oder Erdalkalicarbonaten wie zum Beispiel Natrium-, Magnesium-, Kalium-,Calciumcarbonat bei Temperaturen zwischen 0 °C und dem Siedepunkt des jeweiligen Lösungsmittels, vorzugsweise jedoch zwischen 20 °C und 60 °C, durchgeführten Umsetzungen mit einem Substrat der allgemeinen Formel IV

Nf-L-Fu (IV),

worin Nf für ein Nucleofug wie z.B. Cl, Br, J, CH₃C₆H₄SO₃, oder CF₃SO₃,
L für einen aliphatischen, aromatischen, arylaliphatischen, verzweigten, geradkettigen oder cyclischen Kohlenwasserstoffrest mit bis zu 20 Kohlenstoffatomen und Fu für die gewünschte endständige funktionelle Gruppe, gegebenenfalls in geschützter Form, stehen (DE-OS 34 17 413).

Als Beispiele für Verbindungen der allgemeinen Formel IV seien genannt Br(CH₂)₂NH₂, Br(CH₂)₃OH, BrCH₂COOCH₃, BrCH₂CO₂^{t}Bu, CICH₂CONHNH₂, Br(CH₂)₄CO₂C₂H₅, BrCH₂COBr, ClCH₂COOC₂H₅, BrCH₂CONHNH₂,

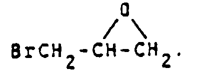

Umwandlungen von Carboxy-Gruppen können zum Beispiel nach der Carbodiimid-Methode (Fieser, Reagents for Organic Syntheses 10,142), über ein gemischtes Anhydrid [Org. Prep. Proc. Int. 7,215(1975)] oder über einen aktivierten Ester (Adv. Org. Chem. Part B. 472) durchgeführt werden.

Die Herstellung der als Ausgangssubstanzen für die Cyclisierung benötigten Amine erfolgt analog literaturbekannten Methoden.

Ausgehend von einer N-geschützten Aminosäure erhält man durch Umsetzung mit einem partiell geschützten Diamin (zum Beispiel nach der Carbadiimidmethode), Abspaltung der Schutzgruppen und Diboranreduktion ein Triamin.

Die Umsetzung eines aus Aminosäuren erhältlichen Diamins (Eur. J. Med. Chem.-Chim. Ther. 21,333 (1986)) mit der doppelten molaren Menge einer N-geschützten ω-Aminosäure liefert ein Tetramin nach geeigneter Aufarbeitung.

In beiden Fällen ist die Anzahl der Kohlenstoffatome zwischen den N-Atomen durch die Art der als Kopplungspartner eingesetzten Diamine bzw. Aminosäuren bestimmbar.

Die Einführung des gewünschten Metalls (Mn) in die Porphyrinliganden erfolgt nach literaturbekannten Methoden (The Porphyrins,ed. D. Dolphin, Academic Press, New York 1980, Vol. V, p. 459) durch Erwärmen mit den entsprechenden Mangansalzen, vorzugsweise den Acetaten, gegebenenfalls unter Zusatz von säurepuffernden Mitteln wie Natriumacetat. Als Lösungsmittel sind vor allem polare Solventien, wie zum Beispiel Chloroform, Essigsäure, Methanol, Ethanol, Dimethylformamid, Wasser geeignet.

Die Komplexierung sollte möglichst unter Lichtausschluß vorgenommen werden, da ein photochemischer Abbau der Porphyrine erfolgen kann.

Enthält die erfindungsgemäße Komplexverbindung den Rest K, so kann die Einführung des Porphyrin-Metalls vor oder nach Anknüpfung des Komplexbildner-Restes K sowie auch vor oder nach Chelatisierung dieses Komplexbildners mit einem Metall erfolgen. Dadurch wird eine besonders flexible Vorgehensweise für die Synthese der erfindungsgemäßen Verbindungen ermöglicht.

Die Chelatisierung des Restes K erfolgt in der Weise, wie sie in der Patentschrift DE-OS 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21-32, 42, 44 oder 57-70 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene azide Wasserstoffatome der Säuregruppen durch Kationen anorganischer und/oder organischer Basen oder Aminosäuren substituiert.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl-und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufallen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie azide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen im Komplex ganz oder teilweise in zum Beispiel Ester oder Amide zu überführen. Dies kann durch nachträgliche Reaktion am fertigen Komplex geschehen (z.B. durch erschöpfende Umsetzung der freien Carboxy- oder Phosphonsäure-Gruppen mit Dimethylsulfat) wie auch durch Verwendung eines geeignet derivatisierten Substrats zur Einführung der Komplexbildner-Einheiten K-V der allgemeinen Formeln I'A, I'B, I'C, I"AB, I"C, (z. B. N³-(2,6-Dioxomorpholinoethyl)-N⁶-(ethoxycarbonylmethyl)-3,6-diataoctandisäure)
Im Falle der Verwendung von Radioisotope enthaltenden Komplexverbindungen kann deren Herstellung nach den in "Radiotracers for Medical Applications", Volume 1 CRC-Press, Boca Raton, Florida beschriebenen Methoden vorgenommen werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 70µMol/L bis 70 m mol/L in Form seines Komplexsalzes und werden in der Regel in Mengen von 0,1 µMol bis 1 mMol/kg Körpergewicht dosiert. Sie sind zur enteralen und parenteralen Applikation bestimmt.

Die erfindungsgemäßen Komplexverbindungen kommen zur Anwendung für die NMR- Diagnostik in Form ihrer Komplexe mit den Ionen der Elemente mit den Ordnungszahlen 21-29, 42, 44 und 57-70;

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es, hochkonzentrierte Lösungen herzustellen, damit die Volumenbelastung des Kreislaufs in vertretbaren Grenzen zu halten und die Verdünnung durch Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht konvalent gebundenen - an sich giftigen -Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 5 µMol bis 100 µMol/kg Körpergewicht, vorzugsweise 50 µMol bis 100 µMol/kg Körpergewicht, dosiert. Details der Anwendung werden zum Beispiel in H. J. Weinmann et al., Am. J. of Roentgenology 142, 619 (1984) diskutiert.
Besonders niedrige Dosierungen (unter 1 mg/kg Körpergewicht) von organspezifischen NMR-Diagnostika sind zum Beispiel zum Nachweis von Tumoren und von Herzinfarkt einsetzbar.
Ferner können die erfindungsgemäßen Komplexverbindungen vorteilhaft als Suszeptibilitäts- Reagenzien und als shift-Reagenzien für die in-vivo NMR-Spektroskopie verwendet werden.

Insgesamt ist es gelungen, neue Komplexverbindungen zu synthetisieren, die neue Möglichkeiten in der diagnostischen Medizin erschließen.

Die nachfolgenden Beispiele dienen der näheren Erläuterung des Erfindungsgegenstandes.

### BEISPIEL 1

### meso-Tetrakis{4-[1-carboxylato-2,5,8-tris(carboxylatomethyl)-2,5,8-triazanonyl carbonylamino]-phenyl}-porphyrin-Gadolinium(III)-komplex, Tetranatriumsalz

### a) N³-(2,6-Dioxomorpholinoethyl)-N⁶-(ethoxycarbonylmethyl)-3,6-diazaoctandisäure

Eine Suspension von 21,1 g (50 mmol) N³,N⁶-Bis-(carboxymethyl)-N⁹-(ethoxycarbonylmethyl)-3,6,9-triazaundecandisäure (J. Pharm. Sci. 68, 1979, 194) in 250 ml Essigsäureanhydrid läßt man nach Zugabe von 42,2 ml Pyridin drei Tage bei Raumtemperatur rühren. Dann saugt man den Niederschlag ab, wäscht ihn dreimal mit je 50 ml Essigsäureanhydrid und verrührt ihn anschließend mehrere Stunden mit absolutem Diethylether. Nach Absaugen, Waschen mit absolutem Diethylether und Trocknen im Vakuum bei 40°C erhält man 18,0 g (89 % der Theorie) eines weißen Pulvers vom Schmelzpunkt 195-196°C.

| Analyse | | | |
|---|---|---|---|
| Ber.: | C 47,64 | H 6,25 | N 10,42 |
| Gef.: | C 47,54 | H 6,30 | N 10,22 |

b) Eine Lösung von 820,6 mg (1 mmol) meso-Tetra-(4-aminophenyl)-porphyrin Tetra hydrochlorid (A.S. Semeikin et al, C.A.165,1936, 133611 h) in einem Gemisch aus 75 ml Dioxan und 75 ml destilliertem Wasser wird mit Natronlauge auf einen pH-Wert von 9 eingestellt. Eine leichte Trübung der Lösung wird durch Zusatz von 10 ml Dioxan behoben. Bei 5°C werden anteilweise 2,42 g (6 mmol) des nach Beispiel 5a) erhaltenen Diethylentriaminpentaessigsäure-ethylestermonoanhydrids zugegeben,wobei der pH-Wert zwischen 8-9 gehalten wird. Nach beendeter Zugabe wird 30 Minuten nachgerührt. Dann stellt man die Lösung auf einen pH-Wert von 10 und läßt über Nacht stehen. Die Lösung wird nun auf einen pH-Wert von 8 gebracht. Durch anteilweise Zugabe von 2,88 g (6 mmol) Gadoliniumtriacetat Tetrahydrat erhält man den Gadoliniumkomplex. Man läßt eine Stunde nachrühren, engt die Lösung im Vakuum ein, nimmt in destilliertem Wasser auf und zentrifugiert. Der Rückstand wird mit Wasser aufgeschlämmt und erneut zentrifugiert. Die vereinigten Lösungen werden im Vakuum zur Trockne eingeengt. Der Rückstand wird der Säulenchromatographie an Kieselgel unterworfen. Das Produkt wird mit einem Dioxan/Ammoniak-Gemisch eluiert, das Eluat dann zur Trockne eingeengt, in Wasser aufgenommen, zentrifugiert und gefriergetrocknet. Das dunkle Pulver wird mit warmem DMF digeriert, abgesaugt, mit Diethylether gewaschen und im Vakuum bei 50°C getrocknet. Ausbeute: 1,17 g (40,6 % der Theorie)

| Analyse: Der Gadoliniumgehalt wird durch AAS bestimmt. | | | | | | |
|---|---|---|---|---|---|---|
| Ber.: | C 41,69 | H 3,75 | N 9,72 | Gd 21,83 | Na 3,19 | O 19,99 |
| Gef.: | C 41,69 | H 3,62 | N 9,68 | Gd 21,75 | Na 3,15 | |

### BEISPIEL 2

### Mangan(III)-{meso-tetrakis-[4-(1-carboxylato-2,5,8-tris(carboxylatomethyl)2,5,8 -triazanonyl-carbonylamino]-phenyl]-porphyrin}-acetat, Gadoliniumkomplex, Tetranatriumsalz

In einem Gemisch aus 10 ml Eisessig und 1 ml destilliertem Wasser werden 266 mg (0,092 mmol) der in Beispiel 1 hergestellten Verbindung gelöst. Nach Zugabe von 16 mg (0,190 mmol) wasserfreiem Natriumacetat erwärmt man auf 80°C, versetzt mit 47 mg (0,190 mmol) Mangandiacetat Tetrahydrat und rührt 2 Stunden. Im Dünnschichtchromatogramm ist kein Ausgangsmaterial mehr erkennbar. Die erhaltene Lösung wird im Vakuum zur Trockne eingeengt. Man nimmt den Rückstand in Wasser auf, stellt alkalisch, zentrifugiert und reinigt das Produkt durch Chromatographie an Kieselgel RP. Die vereinigten Lösungen werden im Vakuum zur Trockne eingeengt. Der Rückstand wird in Wasser gelöst und der Gefriertrocknung unterworfen. Die Titelverbindung wird als grüner Schaum erhalten. Ausbeute: 238 mg (86,4 % der Theorie)

Die Metallbestimmung wird mit dem Plasma Quad der Firma VG Instruments, England vorgenommen.

| | | | | | | | | |
|---|---|---|---|---|---|---|---|---|
| Analyse: | Ber.: | C 40,93 | H 3,47 | N 9,36 | Mn 1,84 | Gd 21,02 | Na 3,07 | O 20,37 |
| | Gef.: | C 40,88 | H 3,52 | N 9,31 | Mn 1,83 | Gd 20,94 | Na 3,03 | |

### BEISPIEL 3

### meso-Tetrakis{-4-[1-carboxylato-2,5,8-tris(carboxylatomethyl)-2,5,8,-triazanonylcarbonylaminol-phenyl}-porphyrin, Ytterbiumkomplex, Tetranatriumsalz

### a) meso-Tetrakis{-4-[1-carboxylato-2,5,8-tris(carboxylatomethyl)-2,5,8,-triaza nonylcarbonylamino]-phenyl}-porphyrin, Hexadecanatriumsalz

Eine Lösung von 820,6 mg (1 mmol) meso-Tetra-(4-aminophenyl)-porphyrin Tetrahydrochlorid (A.S. Semeikin et al., C.A. 105, 1986, 133611h) in einem Gemisch aus 75 ml Dioxan und 75 ml destilliertem Wasser wird mit Natronlauge auf einen pH-Wert von 9 eingestellt. Die Lösung wird auf 5°C gekühlt und unter Rühren werden anteilweise 2,42 g (6 mmol) Diethylentriamin-pentaessigsäure-ethylester-monoanhydrid (Beispiel 1a) zugegeben, wobei der pH-Wert zwischen 8-9 gehalten wird. Man rührt nach beendeter Zugabe noch 30 Minuten nach. Dann stellt man einen pH-Wert von 10 ein und läßt über Nacht rühren. Die Lösung wird der Säulenchromatographie an Kieselgel RP 18 unterworfen und das Produkt mit Methanol eluiert. Die Lösung engt man im Vakuum zur Trockne ein, nimmt den Rückstand in Wasser auf und gewinnt die Titelverbindung durch Gefriertrocknung.

| Ausbeute: 1,754 g (69,4% der Theorie) | | | | | | |
|---|---|---|---|---|---|---|
| Analyse: | Ber.: | C 47,51 | H 4,07 | N 11,08 | Na 14,55 | O 22,79 |
| | Gef.: | C 47,40 | H 4,12 | N 11,00 | Na 14,59 | |

### b) meso-Tetrakis{-4-[1-carboxylato-2,5,8-tris(carboxylatomethyl)-2,5,8,-triaza 4-[nonylcarbonylamino]-phenyl}-porphyrin, Ytterbiumkomplex, Tetranatriumsalz

In 100 ml destilliertem Wasser werden 1,264 g (0,5 mmol) des unter Beispiel 3a) hergestellten Komplexbildners gelöst und unter Rühren anteilweise mit 844,5 mg (2 mmol) Ytterbiumacetat Tetrahydrat versetzt, wobei der pH-Wert zwischen 7-7,5 durch Zugabe von Natronlauge gehalten wird. Man läßt über Nacht nachrühren und unterwirft die Lösung der Säulenchromatographie an Kieselgel RP 18. Die produkthaltigen Fraktionen werden vereinigt, im Vakuum eingeengt und der Rückstand in Wasser gelöst. Die Titelverbindung gewinnt man durch Gefriertrocknung.
Ausbeute: 2,609 g (88,6% der Theorie)

Der Metallgehalt wird mit dem Plasma Quad der Firma VG/England bestimmt.

| | | | | | | | |
|---|---|---|---|---|---|---|---|
| Analyse: | Ber.: | C 40,80 | H 3,49 | Yb 23,51 | N 9,51 | Na 3,12 | O 19,56 |
| | Gef.: | C 40,76 | H 3,52 | Yb 23,48 | N 9,47 | Na 3,10 | |

Man kühlt unter Feuchtigkeitsausschluß auf -10°C und versetzt unter Rühren mit 3,714 mg (18 mmol) Dicyclohexylcarbodiimid. Es wird eine Stunde bei der tiefen Temperatur gerührt, dann läßt man auf Raumtemperatur kommen und verfolgt die Umsetzung dünnschichtchromatographisch, bis kein Ausgangsamin mehr nachzuweisen ist. Man saugt durch eine Fritte, engt das Filtrat im Vakuum ein und verteilt den Rückstand zwischen Natriumbicarbonatlösung und Dichlormethan. Die organische Lösung wird über Natriumsulfat getrocknet und eingeengt.

### BEISPIEL 4

### meso-Tetrakis{4-[1-carboxylato-10-oxo-2,5,8-tris(carboxylatomethyl)-2,5,8,11-tetraazatridecylcarbamoyl]-phenyl}-porphyrin, Gadoliniumkomplex, Tetranatriumsalz

### a) 5,10,15-Tris{4-[2-(t-butyloxycarbonylamino)-ethylcarbamoyl]-phenyl}-20-(4-carboxylatophenyl)-porphyrin, Natriumsalz und meso-Tetrakis{-4-[2-(t-butyloxycarbonylamino)-ethylcarbamoyl]-phenyl}-porphyrin

In 400 ml Dimethylformamid werden 4,74 g (6 mmol) meso-Tetra(4-carboxyphenyl)-porphyrin gelöst und mit 2,757 g (18 mmol) Hydroxybenztriazolhydrat, 2,884 g (18 mmol) N-t-Butyloxycarbonylethylendiamin versetzt von 5.45 einfügen. Das Rohprodukt wird durch Säulenchromatographie an Kieselgel gereinigt. Als Elutionsmittel dient Isopropanol. Aus dem Vorlauf wird durch Einengen im Vakuum das Tetrasubstitutionsprodukt und aus den polareren Fraktionen das Trisubstitutionsprodukt gewonnen:
meso-Tetrakis{-4-[2-(t-butyloxycarbonylamino)-ethylcarbamoyl]-Phenyl}-porphyrin

| Ausbeute: 1,819 g (26,7% der Theorie) | | | | | |
|---|---|---|---|---|---|
| Analyse: | Ber.: | C 71,32 | H 4,92 | N 11,09 | O 12,67 |
| | Gef.: | C 71,24 | H 4,99 | N 11,02 | |

5,10,15-Tris{4-[2-(t-butyloxycarbanylamino)-ethylcarbamoyl]phenyl}-20-(4-carboxylatophenyl)-porphyrin, Natriumsalz

| Ausbeute: 3,914 g (48,7% der Theorie) | | | | | | |
|---|---|---|---|---|---|---|
| Analyse: | Ber.: | C 69,95 | H 4,74 | N 10,46 | Na 1,72 | O 13,14 |
| | Gef.: | C 70,01 | H 4,81 | N 10,49 | Na 1,70 | |

757,8 mg (0,5 mmol) des unter 4a) hergestellten Tetramids werden in 40 ml 2 molarer Salzsäure in Eisessig gelöst und bei Raumtemperatur 2 Stunden belassen. Man engt im Vakuum zur Trockne ein, destilliert Reste von Essigäure azeotrop mit destilliertem Wasser ab und nimmt den Rückstand in 100 ml Wasser auf. Man stellt mit Natronlauge einen pH-Wert von 9 ein, kühlt auf 0°C und versetzt anteilweise mit 1,008 g (2,5 mmol) DTPA-Monoanhydrid (Beispiel 1a), wobei der pH-Wert durch Zugabe von Natronlauge konstant gehalten wird. Man läßt eine Stunde nachrühren, bringt dann auf einen pH-Wert von 10 und läßt über Nacht stehen. Dann senkt man durch Zugabe von Salzsäure den pH-Wert auf 7,5 ab und gibt anteilweise 1,016 g (2,5 mmol) Gadoliniumacetat Tetrahydrat hinzu. Man läßt über Nacht nachrühren und reinigt den Komplex durch Säulenchromatographie an Kieselgel RP 18. Die Titelverbindung wird mit Methanol/Ammoniak eluiert. Man engt die produkthaltigen Fraktionen im Vakuum zur Trockne ein und nimmt in Wasser auf. Die Titelverbindung wird durch Gefriertrocknung erhalten.

| Ausbeute: | 1,174 g (74,3% der Theorie) | | | | | | |
|---|---|---|---|---|---|---|---|
| Analyse: | Ber.: | C 42,55 | H 3,76 | Gd 19,90 | N 10,63 | Na 2,91 | O 20,24 |
| | Gef.: | C 42,60 | H 3,81 | Gd 19,89 | N 10,58 | Na 2,87 | |

### BEISPIEL 5

### Mangan(III)-{meso-tetrakis[4-(1-carboxylato-10-oxo-2,5,8-tris(carboxylatomethyl)-2,5,8,11-tetraazatridecylcarbamoyl)-phenyl]-porphyrin}-acetat, Gadoliniumkomplex, Tetranatriumsalz

In ein Gemisch aus 63 ml Eisessig und 7 ml Wasser werden 656,2 mg (8 mmol) wasserfreies Natriumacetat gegeben und man erwärmt auf 80°C. Dann gibt man 1,581 g (0.5 mmol) des unter 4) hergestellten Gadoliniumkomplexes sowie 1,961 g (8 mmol) Mangan(II)-acetat-tetrahydrat zu und rührt unter Lichtausschluß bei 80-90°C bis im Dünnschichtchromatogramm kein Ausgangsmaterial mehr nachweisbar ist. Die Lösung wird nach dem Abkühlen im Vakuum zur Trockne eingeengt und der Rückstand in Wasser aufgenommen. Die Titelverbindung wird nach Chromatographie an Kieselgel RP gereinigt. Das Produkt wird durch Gefriertrocknung der wäßrigen Lösung erhalten.

| Ausbeute: | 1,311 g (80% der Theorie) | | | | | | | |
|---|---|---|---|---|---|---|---|---|
| Analyse: | Ber.: | C 41,83 | H 3,66 | Gd 19,22 | Mn 1,68 | N 10,27 | Na 2,81 | O 20,53 |
| | Gef.: | C 41,78 | H 3,71 | Gd 19,19 | Mn 1,67 | N 10,25 | Na 2,77 | |

### BEISPIEL 6

### meso-Tetrakis-{-3-[1-carboxylato-2,5,8-tris-(carboxylatomethyl)-2,5,8-triazanonylcarbonylamino]-4-methylphenyl}-porphyrin, Gadoliniumkomplex, Tetranatriumsalz

### a) meso-Tetrakis-(4-methyl-3-nitrophenyl)-porphyrin

In 50 ml konzentrierter Schwefelsäure werden 1,342 g (2 mmol) 5,10,15,20-(4-Methylphenyl)-porphyrin (A.S. Semeikin et al.,Klim. Geterotsikl. Soedin. 1986, 6, 798) gelöst. Zu der auf 0°C gekühlten Lösung werden unter Rühren 864 mg (9,6 mmol) 70%ige Salpetersäure so zugetropft, daß die Temperatur gehalten wird. Man läßt 2 Stunden bei 0°C und dann weitere 2 Stunden bei Raumtemperatur rühren. Dann gießt man auf 500 ml Eiswasser, neutralisiert mit Natronlauge und filtriert den Feststoff ab. Man wäscht mit Wasser, trocknet im Vakuum und erhält so die Titelverbindung als dunkles Pulver.

| Ausbeute: 1,152 g (67,7% der Theorie) | | | | | |
|---|---|---|---|---|---|
| Analyse: | Ber.: | C 67,76 | H 4,03 | N 13,17 | O 15,04 |
| | Gef.: | C 67,72 | H 4,08 | N 13,13 | |

### b) meso-Tetrakis-[3-amino-4-methylporphyrin]

In 300 ml konzentrierter Salzsäure werden 5,352 g (6,29 mmol) der unter 6a) hergestellten Nitroverbindung suspendiert und auf 70-80°C erwärmt. Unter Rühren werden nun innerhalb von 30 Minuten 30,0 g (133 mmol) Zinn(II)-chlorid Dihydrat zugegeben. Man läßt abkühlen, versetzt mit 300 ml Wasser, zentrifugiert den Niederschlag ab, wäscht mit halbkonzentrierter Salzsäure nach, löst den Niederschlag in 2 Liter Wasser, filtriert und neutralisiert die grüne Lösung mit Ammoniak. Der ausgefallene Feststoff wird mit Wasser gewaschen. Man löst den Feststoff in Ethanol und reinigt durch Säulenchromatographie an Kieselgel. Als Elutionsmittel dient Dioxan/wäßriger Ammoniak. Die produkthaltigen Fraktionen werden im Vakuum zur Trockne eingeengt, in Ethanol aufgenommen, filtriert und erneut zur Trockne eingeengt. Man erhält die Titelverbindung als Schaum.

| Ausbeute: 3,168 g (68,9% der Theorie) | | | | |
|---|---|---|---|---|
| Analyse: | Ber.: | C 78,88 | H 5,79 | N 15,35 |
| | Gef.: | C 78,80 | H 5,86 | N 15,30 |

### c) meso-Tetrakis-{3-[1-carboxylato-2,5,8-tris-(carboxylatomethyl)-2,5,8-triazanonylcarbonylamino]-4-methylphenyl}-porphyrin, Gadoliniumkomplex, Tetranatriumsalz

In Analogie zur Vorschrift für Beispiel 1b) werden 730,9 mg (1 mmol) der unter 6b) hergestellten Tetraaminoverbindung in einem Gemisch aus 75 ml Dioxan und 95 ml destilliertem Wasser gelöst und mit 2,42 g (6 mmol) DTPA-Monoanhydrid (Beispiel 5a) umgesetzt. Der so erhaltene Komplexbildner wird mit 2,88 g (6 mmol) Gadoliniumacetat Tetrahydrat in den Gadoliniumkomplex überführt. Aufarbeitung und Reinigung erfolgen wie in Beispiel 5 beschrieben. Die Titelverbindung wird durch Gefriertrocknung der wäßrigen Lösung gewonnen.

| Ausbeute: 1,686 g (57,4% der Theorie) | | | | | | | |
|---|---|---|---|---|---|---|---|
| Analyse: | Ber.: | C 42,53 | H 3,78 | Gd 21,42 | N 9,54 | Na 3,13 | O 19,61 |
| | Gef.: | C 42,48 | H 3,82 | Gd 21,36 | N 9,50 | Na 3,09 | |

### BEISPIEL 7

### Mangan(III)-{meso-tetrakis<3-(1-carboxylato-2,5,8-tris-(carboxylatomethyl)2,5,8-triaazanonylcarbonylmethyl)-4-methylphenyl>-porphyrin}-acetat, Gadoliniumkomplex, Tetranatriumsalz

In Analogie zu Beispiel 2 werden 1,469 g (0,5 mmol) des unter 6c) hergestellten Gadoliniumkomplexes in einem Gemisch aus 60 ml Eisessig und 6 ml destilliertem Wasser mit 205,1 mg (2,5 mmol) wasserfreiem Natriumacetat und 612,7 mg (2,5 mmol) Mangan(II)-acetat Tetrahydrat umgesetzt und aufgearbeitet. Nach Gefriertrocknung der wäßrigen Lösung erhält man die Titelverbindung als Schaum.

| Ausbeute: 1,262 g (82,8% der Theorie) | | | | | | | | |
|---|---|---|---|---|---|---|---|---|
| Analyse: | Ber.: | C 41,76 | H 3,67 | Gd 20,63 | M 1,80 | N 9,19 | Na 3,02 | O 19,94 |
| | Gef.: | C 41,70 | H 3,73 | Gd 20,58 | M 1,79 | N 9,13 | Na 2,99 | |

## Patentansprüche

1. Pharmazeutische Mittel für die NMR-Diagnostik enthaltend mindestens eine Porphyrin-Komplexverbindung bestehend aus einem meso-Tetraphenylporphyrinliganden der allgemeinen Formel I und mindestens einem paramagnetischen Ion eines Elementes der Ordnungszahlen 21-29, 42, 44 oder 57-70 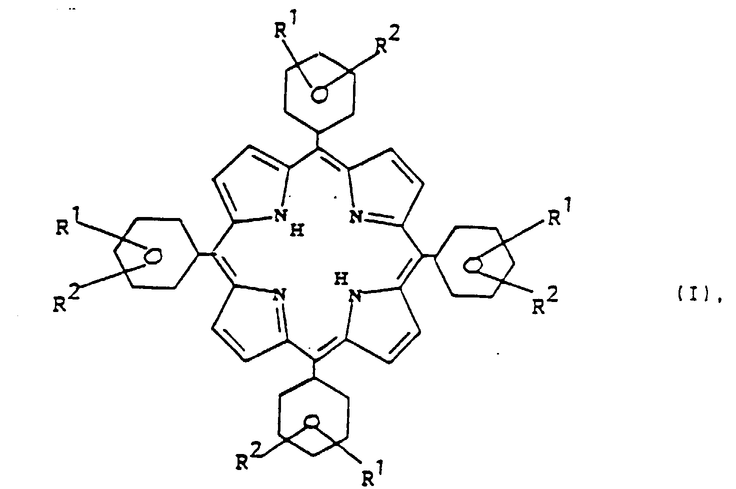 worin
R¹ für einen CO-A-, W- oder NH-W-Rest mit
A in der Bedeutung einer (NH)ₓ-[Q-(NH)_{y}]_{w}-W-Gruppe,
x und y die Ziffern 0, 1 oder 2
w die Ziffern 0 oder 1,
Q eine C₁-C₂₀-Alkylengruppe,
W die Gruppierung V-K bedeutet, wobei V eine gegebenenfalls Imino-, Polyethylenoxy-, Phenylen-, Phenylenoxy-, Phenylenimino-, Amido-, Hydrazido-, Estergruppe(n), Sauerstoff-, Schwefel- und/oder Stickstoff-Atom(e) enthaltende gegebenenfalls durch Hydroxy-, Mercapto-, Imino-, Epoxy-, Oxo-, Thioxo- und/oder Aminogruppe(n) substituierte geradkettige, verzweigte gesättigte oder ungesättigte C₁-C₂₀-Alkylengruppe oder eine direkte Bindung,
K ein Komplexbildner der allgemeinen Formeln IA, IB oder IC 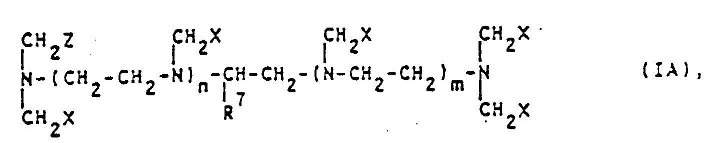 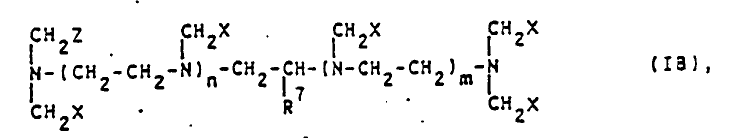 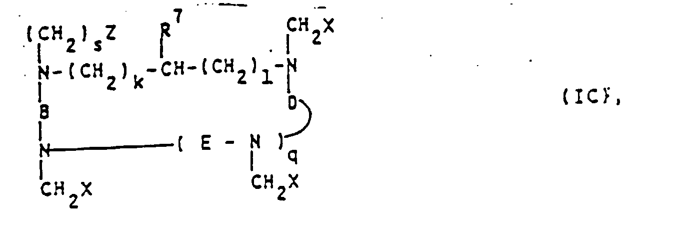
bedeuten,
wobei
n und m jeweils für die Ziffern 0, 1, 2, 3 oder 4, wobei n und m zusammen nicht mehr als 4 ergeben,
k für die Ziffern 1, 2, 3, 4 oder 5,
l für die Ziffern 0, 1, 2, 3, 4 oder 5,
q für die Ziffern 0, 1 oder 2,
s für die Ziffern 0 oder 1,
X für -COOH,
B, D und E, die gleich oder verschieden sind, jeweils für die Gruppe 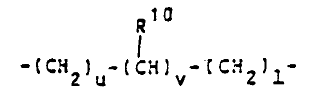 mit
R¹⁰ in der Bedeutung von Wasserstoff oder einer gegebenenfalls Sauerstoff- und/oder Stickstoffatom(e) enthaltenden gegebenenfalls durch Hydroxy-und/oder Aminogruppe(n) substituierten geradkettigen, verzweigten, gesättigten oder ungesätigten C₁-C₂₀-Alkylgruppe.
u in der Bedeutung der Ziffern 0, 1, 2, 3, 4 oder 5,
v in der Bedeutung der Ziffern 0 oder 1,
wobei B, D und E jeweils mindestens 2 und maximal 5 Kahlenstoffatome enthalten,
Z für die Gruppe 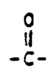 oder den Rest X,
R⁷ für eine direkte Bindung oder ein Wasserstoffatom, stehen,
mit der Maßgabe, daß Z nur dann für die Gruppe 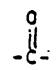 steht, wenn R⁷ gleichzeitig ein Wasserstoffatom bedeutet, und das Z nur dann für den Rest X steht, wenn R⁷ gleichzeitig eine direkte Bindung und s die Ziffer 1 bedeuten.
R² für ein Wasserstoff-, Fluor-, Chlor-, Brom-, Jod-atom oder R⁴, wobei R⁴ einen C₁-C₆-Alkyl - oder Benzylrest bedeutet, steht, wobei gewünschtenfalls noch vorhandene freie Carbonsäuregruppen teilweise oder vollständig als Ester und/oder Amid oder als Salz einer anorganischen und/oder organischen Base, Aminosäure oder Aminosäureamid vorliegen, gegebenenfalls mit den in der Galenik üblichenZusätzen unter der Maßgabe, daß das gegebenenfalls im Porphyrin-Gerüst enthaltene Metall Mangan ist.

2. Paramagnetische Komplexverbindungen, bestehend aus einem meso-Tetraphenylporphyrinliganden der allgemeinen Formel I 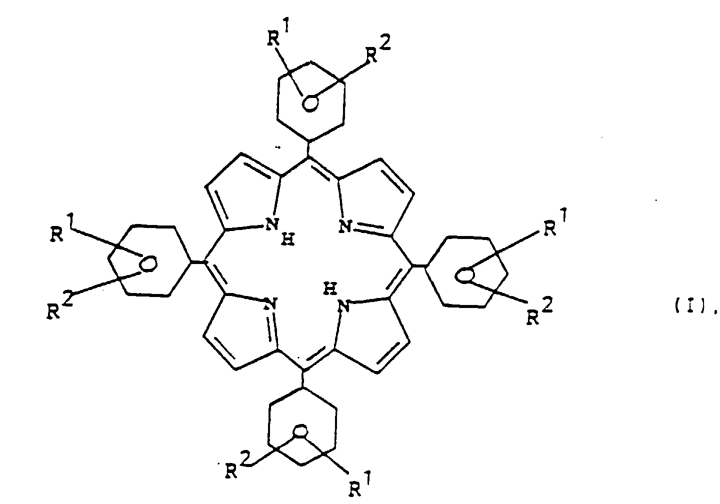 worin die Substituenten die in Anspruch 1 genannten Bedeutungen haben und mindestens einem Ion eines Elements der Ordnungszahlen 21-29, 42, 44, oder 57-70 wobei gewünschtenfalls noch vorhandene freie Carbonsäuregruppen ganz oder teilweise als Ester und/oder Amid oder als Salz einer anorganischen und/oder organischen Base oder Aminosäure oder Aminosäureamid vorliegen, unter der Maßgabe, daß das gegebenenfalls im Porphyringerüst enthaltene Metall Mangan ist.

3. Komplexverbindungen gemäß Anspruch 2, dadurch gekennzeichnet, daß V für eine -(CH₂)₂NH-; -CH₂-O-C₆H₄-CH₂-; -CH₂-CH(OH)-CH₂-O-C₆H₄-CH₂-; -C(=NH)-O-C₆H₄- CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂-; -CH₂-CO-NH-(CH₂)₃-O-CH₂-; -CH₂-CO-NH-HH-; -CH₂-CO-NH- -(CH₂)₂-; -CH₂-CO-NH(CH₂)₁₀-; -CH₂-CO-NH-(CH₂)₂-S-; -(CH₂)₄-NH-CO-(CH₂)₈-; -CH₂-CO-NH-(CH₂)₃-NH-; -(CH₂)₃-NH-; -(CH₂)-NH-C(=S)-NH-C₆H₄-CH₂-; -(CH₂)₂-NH-CO-CH₂-(OCH₂CH₂)₄₃-OCH₂-gruppe steht.

4. Verwendung von mindestens einer Porphyrin-Komplexverbindung gemäß Anspruch 2 für die Herstellung von Mitteln für die NMR-Diagnostik.

5. Verfahren zur Herstellung von meso-Tetraphenylporphyrin-Komplexverbindungen bestehend aus einem Liganden der allgemeinen Formel I und mindestens einem paramagnetischen Ion eines Elementes der Ordnungszahlen 21-29, 42, 44 oder 57-70 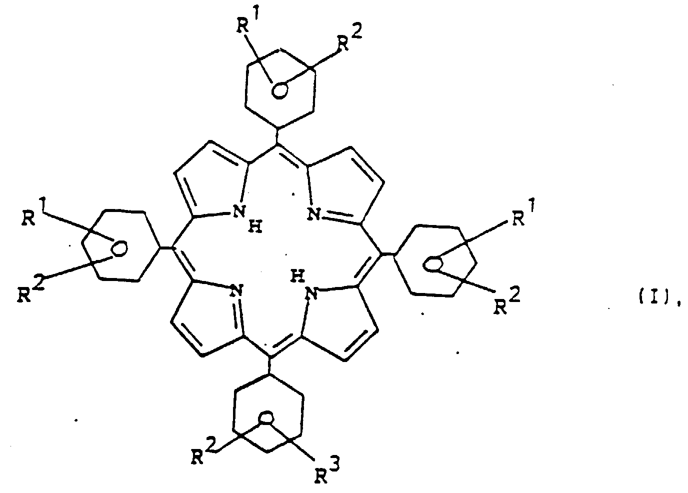 worin
R¹ für einen CO-A-, W- oder NH-W-Rest mit
A in der Bedeutung einer (NH)ₓ-[Q-(NH)_{y}]_{w}-W-Gruppe,
x und y die Ziffern 0, 1 oder 2
w die Ziffern 0 oder 1,
Q eine C₁-C₂₀-Alkylengruppe,
W die Gruppierung V-K bedeutet, wobei V eine gegebenenfalls Imino-, Polyethylenoxy-, Phenylen-, Phenylenoxy-, Phenylenimino-, Amido-, Hydrazido-, Estergruppe(n), Sauerstoff-, Schwefel- und/oder Stickstoff-Atom(e) enthaltende gegebenenfalls durch Hydroxy-, Mercapto-, Imino-, Epoxy-, Oxo-, Thioxo- und/oder Aminogruppe(n) substituierte geradkettige, verzweigte, gesättigte oder ungesättigte C₁-C₂₀- Alkylengruppe oder eine direkte Bindung,
K ein Komplexbildner der allgemeinen Formeln IA, IB oder IC 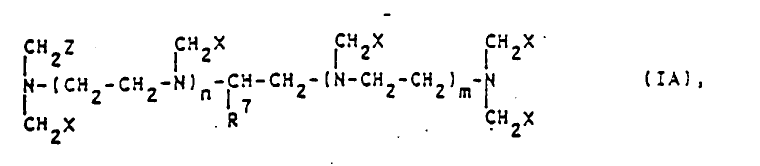 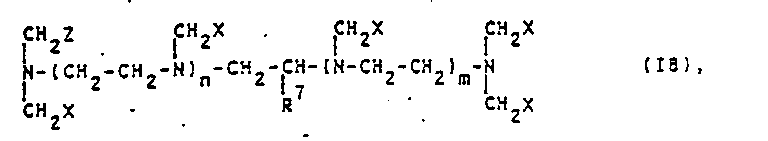 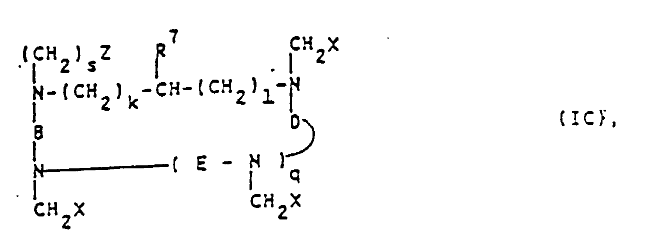
bedeuten,
wobei
n und m jeweils für die Ziffern 0, 1, 2, 3 oder 4, wobei n und m zusammen nicht mehr als 4 ergeben,
k für die Ziffern 1, 2, 3, 4 oder 5,
l für die Ziffern 0, 1, 2, 3, 4 oder 5,
q für die Ziffern 0, 1 oder 2,
s für die Ziffern 0 oder 1,
X für -COOH.
B, D und E, die gleich oder verschieden sind. jeweils für die Gruppe 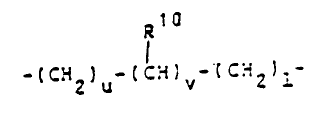 mit
R¹⁰ in der Bedeutung von Wasserstoff oder einer gegebenenfalls Sauerstoff- und/oder Stickstoffatom(e) enthaltenden gegebenenfalls durch Hydroxy-und/oder Aminogruppe(n) substituierten geradkettigen, verzweigten, gesättigten oder ungesättigten C₁-C₂₀-Alkylgruppe,
u in der bedeutung der Ziffern 0, 1, 2, 3, 4 oder 5,
v in der Bedeutung der Ziffern 0 oder 1,
wobei B, D und E jeweils mindestens 2 und maximal 5 Kohlenstoffatome enthalten,
Z für die Gruppe 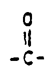 oder den Rest X,
R⁷ für eine direkte Bindung oder ein Wasserstoffatom, stehen,
mit der Maßgrabe, daß Z nur dann für die Gruppe 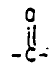 steht, wenn R⁷ gleichzeitig ein Wasserstoffatom bedeutet, und daß Z nur dann für den Rest X steht, wenn R⁷ gleichzeitig eine direkte Bindung und s die Ziffer 1 bedeuten,
R² für ein Wasserstoff-, Fluor-, Chlor-, Brom-, Jod-atom oder R⁴, wobei R⁴ einen C₁-C₆-Alkyl oder Benzylrest bedeutet, steht, wobei hewünschtenfalls noch vorhandene freie Carbonsäuregruppen teilweise oder vollständig als Ester und/oder Amid oder als Salz einer anorganischen und/oder organischen Base Aminosäure oder Aminosäureamid vorliegen, gegebenenfalls mit den in der Galenik üblichenZusätzen unter der Maßgabe, daß das gegebenenfalls im Porphyrin-Gerüst enthaltene Metall Mangan ist, dadurch gekennzeichnet, daß man Porphyrine der allgemeinen Formel II 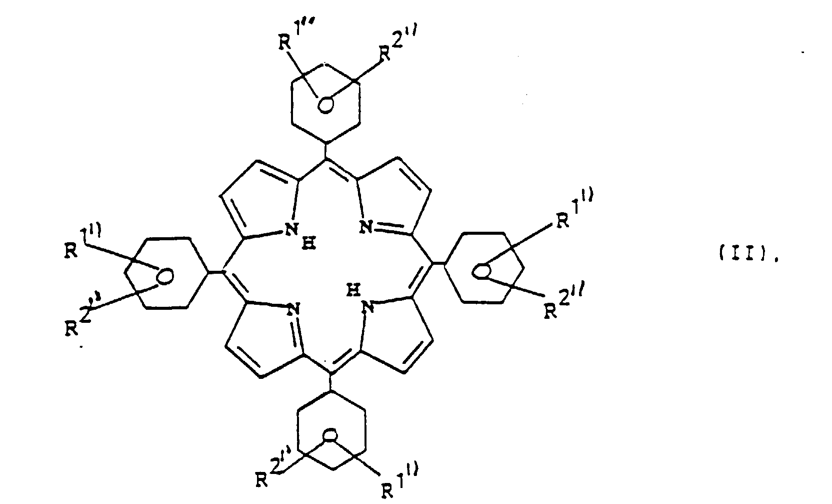
worin
R^{1"} für R^{1'} oder einen Substituenten, der in R^{1'} umgewandelt werden kann,
R^{2"} für ein Wasserstoff-, Fluor-, Chlor-, Brom-, Jod-atom, oder, R⁴
wobei
R^{1'} und R^{2'} die für R¹ und R² angegebene Bedeutung haben, der Substituent
W jedoch für ein Wasserstoffatom steht, gegebenenfalls in an sich bekannter Weise in Porphyrine der allgemeinen Formel II' überführt, worin R^{1"}, R^{2"} für R^{2'} stehen, und anschließend
a) die pyrrolischen NH's durch das Manganatom substituiert, gegebenenfalls vorhandene Schutzgruppen entfernt und anschließend den Rest V-K einführt und danach mit mindestens einem Metalloxid oder Metallsalz eines Elements der Ordnungszahlen 21-29, 42, 44 oder 57-70 umsetzt oder
b) den Substituenten V-K einführt, mit mindestens einem Metalloxid oder Metallsalz eines Elements der Ordnungszahlen 21-29, 42, 44 oder 57-70 umsetzt und anschließend gewünschtenfalls die pyrrolischen NH's durch ein Manganatom substituiert oder
c) den Substituenten V-K einführt, die pyrrolischen NH's durch ein Manganatom substituiert und anschließend mit mindestens einem Metalloxid oder Metallsalz eines Elements der Ordnungszahlen 21-29, 42, 44 oder 57-70 umsetzt und gegebenenfalls anschließend in den nach a), b) oder c) erhaltenen Komplexverbindungen noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester und/oder Amide überführt.

6. Verfahren zur Herstellung der pharmazeutischen Mittel gemäß Anspruch 1, dadurch gekennzeichnet, daß man die in Wasser oder physiologischer Salzlösung gelöste oder suspendierte Porphyrin-Komplexverbindung gegebenenfalls mit den in der Galenik üblichen Zusätzen, in eine für die enterale oder parenterale Applikation geeignete Form bringt.

## Claims

1. Pharmaceutical compositions for NMR diagnostics, containing at least one porphyrin complex compound consisting of a mesa-tetraphenylporphyrin ligand of the general formula I and at least one paramagnetic ion of an element having an atomic number of from 21 to 29, 42, 44 or from 57 to 70 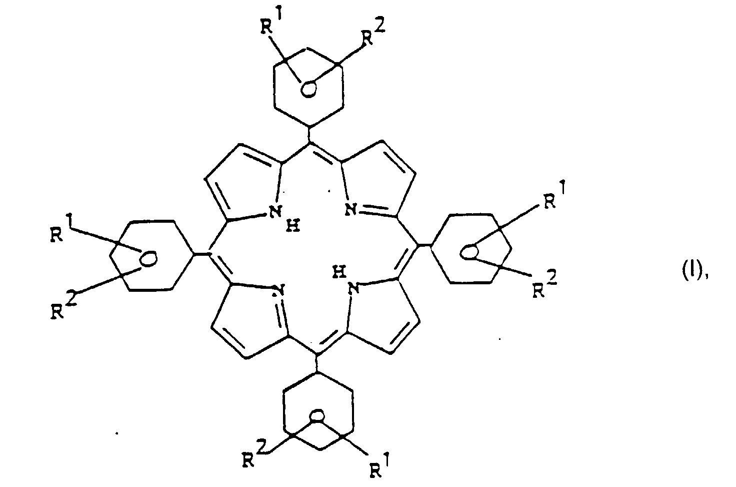 wherein
R¹ represents a CO-A, a W or an NH-W radical wherein A is an (NH)ₓ-[Q-(NH)_{y}]_{w-}W group,
x and y represent the number 0, 1 or 2,
w represents the number 0 or 1,
Q represents a C₁-C₂₀alkylene group,
W represents the grouping V-K wherein V is a straight-chained or branched, saturated or unsaturated C₁-C₂₀alkylene group that is unsubstituted or substituted by hydroxy, mercapto, imino, epoxy, oxo, thioxo and/or amino group(s) and optionally contains imino, polyethyleneoxy, phenylene, phenyleneoxy, phenyleneimino, amido, hydrazido and/or ester group(s), and/or oxygen, sulphur and/or nitrogen atom(s), or is a direct bond,
K represents a complex former of the general formula IA, IB or IC 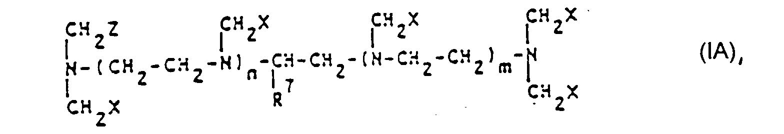 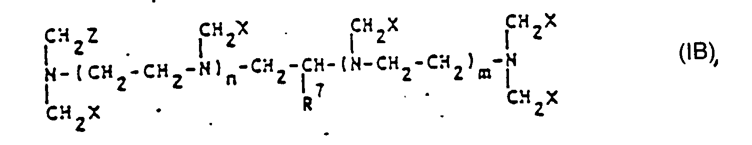 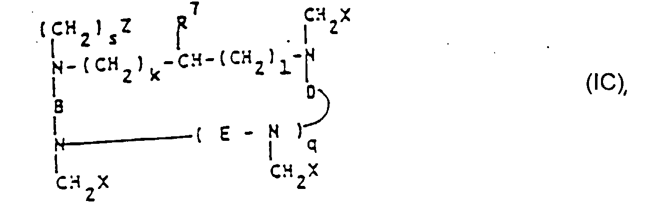
wherein
n and m each represent the number 0, 1, 2, 3 or 4, wherein the total of n and m does not exceed 4,
k represents the number 1, 2, 3, 4 or 5,
I represents the number 0, 1, 2, 3, 4 or 5,
q represents the number 0, 1 or 2,
s represents the number 0 or 1,
X represents -COOH,
B, D and E, which are identical or different, each represent the group 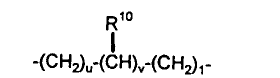
wherein
R¹⁰ is hydrogen or a straight-chained or branched, saturated or unsaturated C₁-C₂₀alkyl group that is unsubstituted or substituted by hydroxy and/or amino group(s) and optionally contains oxygen and/or nitrogen atom(s),
u is the number 0, 1, 2, 3, 4 or 5,
v is the number 0 or 1,
B, D and E each containing a minimum of 2 and a maximum of 5 carbon atoms,
Z represents the group 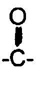 or the radical X,
R⁷ represents a direct bond or a hydrogen atom,
with the proviso that Z represents the group 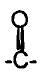 only when R⁷ simultaneously represents a hydrogen atom and that Z represents the radical X only when R⁷ simultaneously represents a direct bond and s represents the number 1,
R² represents a hydrogen, fluorine, chlorine, bromine or iodine atom or R⁴,
R⁴ representing a C₁-C₆alkyl or benzyl radical,
if desired any free carboxylic acid groups that are still present being partially or totally in the form of ester and/or amide or as salt of inorganic and/or organic base, amino acid or amino acid amide, optionally together with the additives customary in galenical pharmacy, with the proviso that the metal that is optionally present in the porphyrin structure is manganese.

2. Paramagnetic complex compounds consisting of a mesa-tetraphenylporphyrin ligand of the general formula I 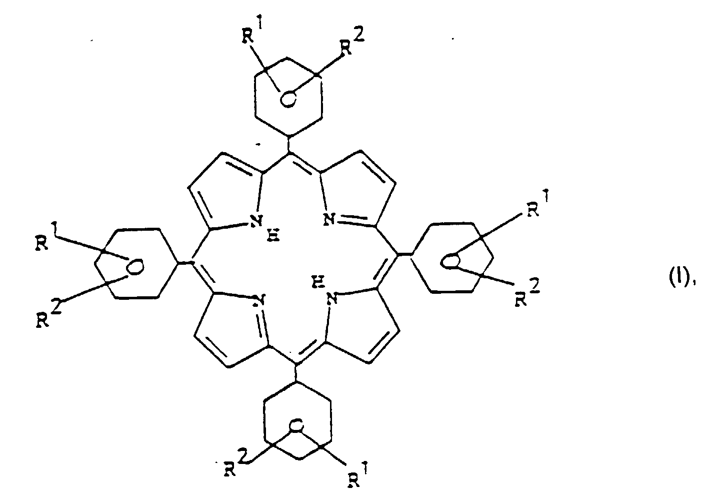 wherein the substituents have the meanings given in claim 1, and at least one ion of an element having a atomic number of from 21 to 29, 42, 44 or from 57 to 70, if desired any free carboxylic acid groups that are still present being totally or partially in the form of ester and/or amide or as salt of inorganic and/or organic base or amino acid or amino acid amide, with the proviso that the metal that is optionally present in the porphyrin structure is manganese.

3. Complex compounds according to claim 2, characterised in that V is a -(CH₂)₂NH-; -CH₂-O-C₆H₄-CH₂-; -CH₂-CH(OH)-CH₂-O-C₆H₄-CH₂-; -C(=NH)-O-C₆H₄-CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂-; -CH₂-CO-NH-(CH₂)₃-O-CH₂-; -CH₂-CO-NH-NH-; -CH₂-CO-NH-(CH₂)₂-; -CH₂-CO-NH(CH₂)₁₀-; -CH₂-CO-NH-(CH₂)₂-S-; -(CH₂)₄-NH-CO-(CH₂)₈-; -CH₂-CO-NH-(CH₂)₃-NH-; -(CH₂)₃-NH-; -(CH₂)-NH-C(=S)-NH-C₆H₄-CH₂- or -(CH₂)₂-NH-CO-CH₂-(OCH₂CH₂)₄₃-OCH₂- group.

4. Use of at least one porphyrin complex compound according to claim 2 in the preparation of compositions for NMR diagnostics.

5. Process for the preparation of mesa-tetraphenylporphyrin complex compounds consisting of a ligand of the general formula I and at least one paramagnetic ion of an element having an atomic number of from 21 to 29, 42, 44 or from 57 to 70 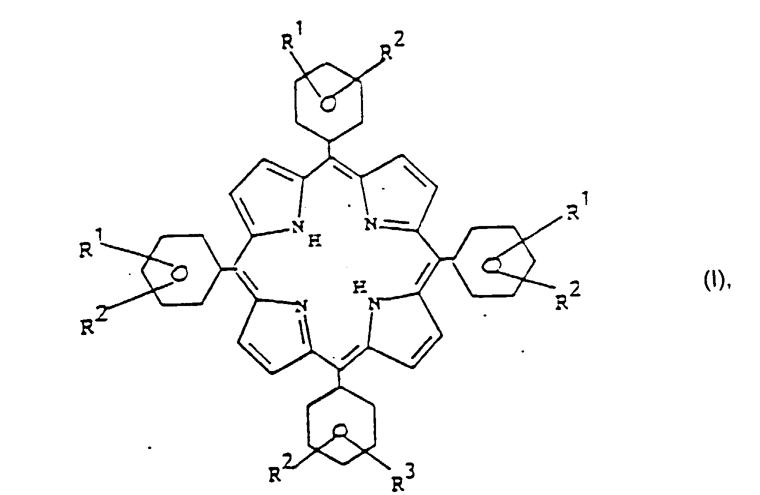 wherein
R¹ represents a CO-A, a W or an NH-W radical wherein A is an (NH)ₓ-(Q-(NH)_{y}]_{w-}W group,
x and y represent the number 0, 1 or 2,
w represents the number 0 or 1,
Q represents a C₁-C₂₀alkylene group,
W represents the grouping V-K wherein V is a straight-chained or branched,
saturated or unsaturated C₁-C₂₀alkylene group that is unsubstituted or substituted by hydroxy, mercapto, imino, epoxy, oxo, thioxo and/or amino group(s) and optionally contains imino, polyethyleneoxy, phenylene, phenyleneoxy, phenyleneimino, amido, hydrazido and/or ester group(s), and/or oxygen, sulphur and/or nitrogen atom(s), or is a direct bond,
K represents a complex former of the general formula IA, IB or IC 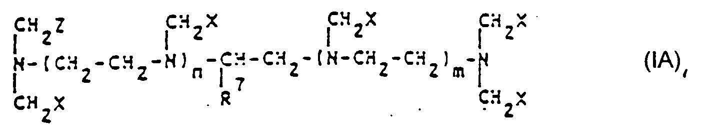 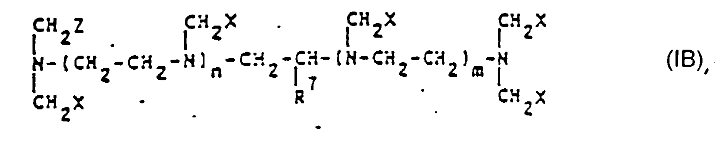 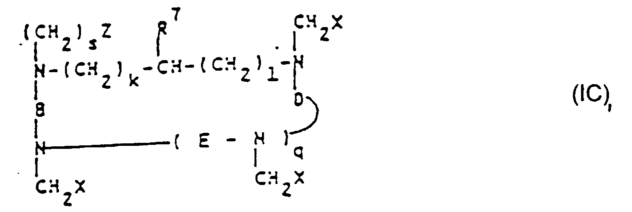
wherein
n and m each represent the number 0, 1, 2, 3 or 4, wherein the total of n and m does not exceed 4,
k represents the number 1, 2, 3, 4 or 5,
I represents the number 0, 1, 2, 3, 4 or 5,
q represents the number 0, 1 or 2,
s represents the number 0 or 1,
X represents -COOH,
B, D and E, which are identical or different, each represent the group 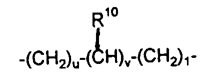
wherein
R¹⁰ is hydrogen or a straight-chained or branched, saturated or unsaturated C₁-C₂₀alkyl group that is unsubstituted or substituted by hydroxy and/or amino group(s) and optionally contains oxygen and/or nitrogen atom(s),
u is the number 0, 1, 2, 3, 4 or 5,
v is the number 0 or 1,
B, D and E each containing a minimum of 2 and a maximum of 5 carbon atoms,
Z represents the group 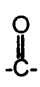 or the radical X,
R⁷ represents a direct bond or a hydrogen atom,
with the proviso that Z represents the group 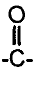 only when R⁷ simultaneously represents a hydrogen atom and that Z represents the radical X only when R⁷ simultaneously represents a direct bond and s represents the number 1,
R² represents a hydrogen, fluorine, chlorine, bromine or iodine atom or R⁴,
R⁴ representing a C₁-C₆alkyl or benzyl radical,
if desired any free carboxylic acid groups that are still present being partially or totally in the form of ester and/or amide or as salt of inorganic and/or organic base, amino acid or amino acid amide, optionally together with the additives customary in galenical pharmacy, with the proviso that the metal that is optionally present in the porphyrin structure is manganese, characterised in that porphyrins of the general formula II 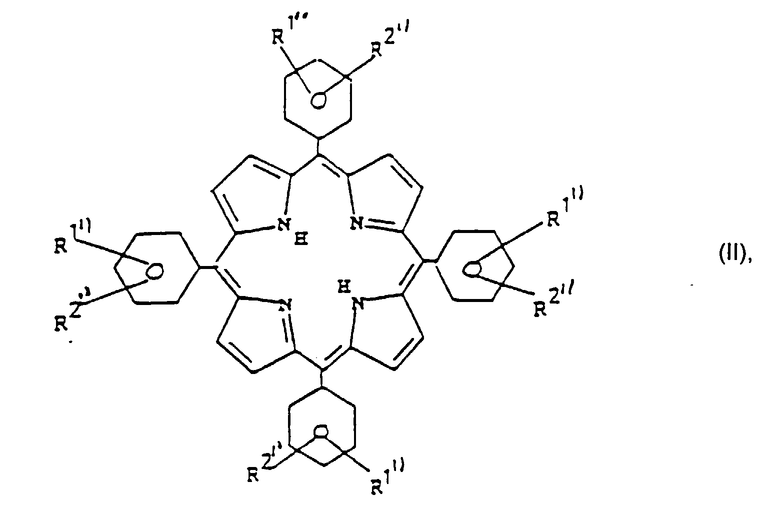
wherein
R^{1"} represents R^{1'} or a substituent that can be converted into R^{1'},
R^{2"} represents a hydrogen, fluorine, chlorine, bromine or iodine atom or R⁴, wherein
R^{1'} and R^{2'} have the meaning given for R¹ and R², but the substituent W is a hydrogen atom,
are optionally converted in a manner known *per se* into porphyrins of the general formula II', wherein R^{1"} and R^{2"} represent R^{2'}, and then
a) the pyrrolic NHs are substituted by the manganese atom, any protecting groups that are present are removed, then the radical V-K is introduced and then the compound is reacted with at least one metal oxide or metal salt of an element having an atomic number of from 21 to 29, 42, 44 or from 57 to 70, or
b) the substituent V-K is introduced, the compound is reacted with at least one metal oxide or metal salt of an element having an atomic number of from 21 to 29, 42, 44 or from 57 to 70, and then, if desired, the pyrrolic NHs are substituted by a manganese atom, or
c) the substituent V-K is introduced, the pyrrolic NHs are substituted by a manganese atom and then the compound is reacted with at least one metal oxide or metal salt of an element having an atomic number of from 21 to 29, 42, 44 or from 57 to 70,
and then, where appropriate, any acid hydrogen atoms that are still present in the complex compounds obtained in accordance with a), b) or c) are replaced by cations of inorganic and/or organic bases, amino acids or amino acid amides and/or the acid groups in question are converted partially or totally into esters and/or amides.

6. Process for the preparation of the pharmaceutical compositions according to claim 1, characterised in that the porphyrin complex compound, dissolved or suspended in water or physiological salt solution, optionally together with the additives customary in galenical pharmacy, are brought into a form suitable for enteral or parenteral administration.

## Revendications

1. Agents pharmaceutiques pour le diagnostic par la RMN contenant au moins un composé complexe de la porphyrine constitué d'un ligand de méso-tétraphénylporphyrine de formule générale I et d'au moins un ion paramagnétique d'un élément ayant un nombre atomique de 21 à 29, 42, 44 ou de 57 à 70 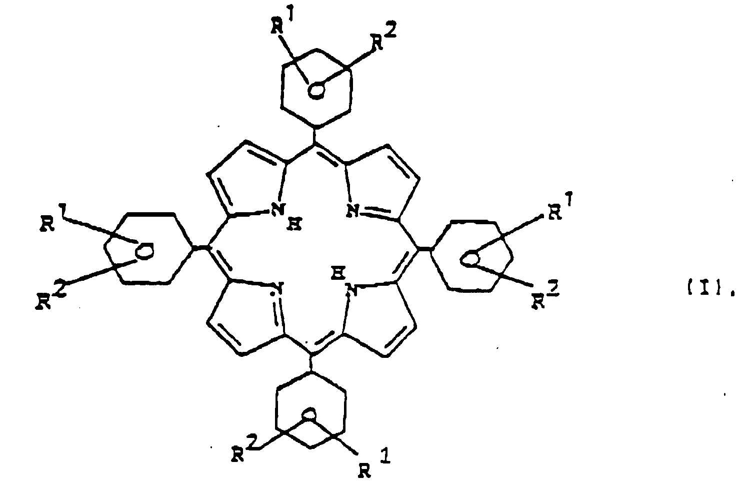 dans laquelle
R¹ représente le radical CO-A, W ou NH-W avec A étant un groupe (NH)ₓ-[Q-(NH)_{y}]_{w}-W
x et y valent 0, 1 ou 2
w vaut 0 ou 1
Q représente un groupe alkylène en C₁-C₂₀,
W représente le groupement V-K, V étant un groupe alkylène en C₁-C₂₀ linéaire, ramifié, saturé ou insaturé, contenant éventuellement un ou des groupe(s) imino, polyéthylènoxy, phénylène, phénylénoxy, phénylénimino, amido, hydrazido, ester, un ou des atome(s) d'oxygène, de soufre et/ou d'azote, éventuellement substitué par un ou des groupe(s) hydroxy, mercapto, imino, époxy, oxo, thioxo et/ou amino contenant un ou des atome(s) d'oxygène ou représente une liaison directe,
K représente un agent complexant de formules générales IA, IB ou IC 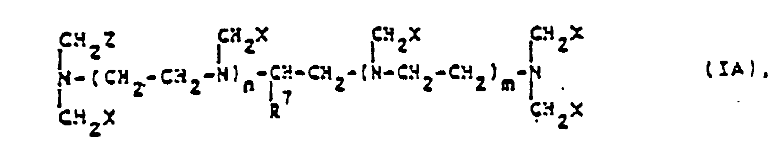 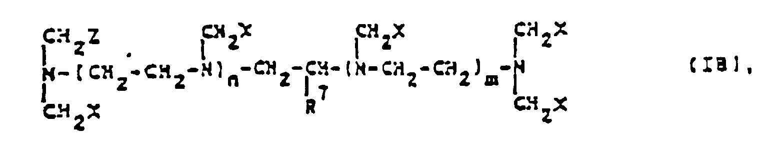 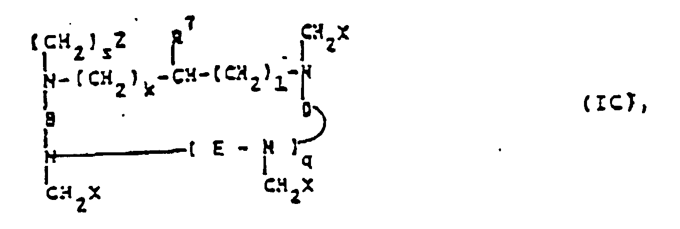 où
n et m valent chacun 0, 1, 2, 3 ou 4, n et m ensemble ne peuvent pas être supérieurs à 4,
k vaut 1, 2, 3, 4 ou 5,
l vaut 0, 1, 2, 3, 4 ou 5,
q vaut 0, 1 ou 2,
s vaut 0 ou 1,
X représente -CODE
B, D et E, qui sont identiques ou différents, représentent chacun le groupe 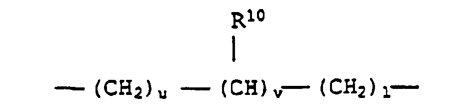 avec
R¹⁰ étant l'hydrogène ou un groupe alkyle en C₁-C₂₀ linéaire, ramifié, saturé ou insaturé, éventuellement contenant un ou des atome(s) d'oxygène et/ou d'azote, éventuellement substitué par un ou des groupe(s) hydroxyle et/ou amino,
u vaut 0, 1, 2, 3, 4 ou 5
v vaut 0 ou 1
B, D et E chacun contenant au moins 2 et au maximum 5 atomes de carbone,
Z représente le groupe 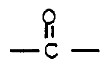 ou le radical X,
R⁷ représente une liaison directe ou un atome d'hydrogène,
à la condition que Z ne soit le groupe 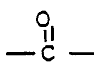 que si R⁷ représente simultanément un atome d'hydrogène, et que Z ne représente le radical X que si R⁷ représente simultanément une liaison directe et s le nombre 1,
R² représente un atome d'hydrogène, de fluor, de chlore, de brome, d'iode ou R⁴, R⁴ étant un alkyle en C₁-C₆ ou un radical benzyle, si on le souhaite, les groupes carboxyles libres encore présents étant partiellement ou entièrement sous la forme d'ester et/ou d'amide ou de sel d'une base organique et/ou inorganique, d'amino-acide ou d'amide d'amino-acide, éventuellement comportant des additifs usuels en galénique, à la condition que le métal éventuellement contenu dans la charpente de la porphyrine soit le manganèse.

2. Composés complexes paramagnétiques constitués d'un ligand méso-tétraporphyrine de formule générale I 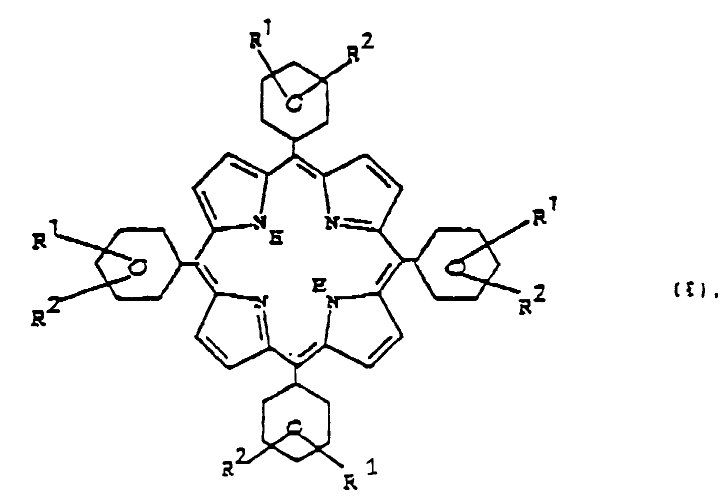 dans laquelle les substituants ont les significations données dans la revendication 1, et d'au moins un ion d'un élément ayant un nombre atomique de 21 à 29, 42, 44 ou de 57 à 70, si on le souhaite, les groupes carboxyles libres encore présents étant partiellement ou entièrement sous la forme d'ester et/ou d'amide ou de sel d'une base organique et/ou inorganique ou d'amino-acide ou d'amide d'amino-acide, à la condition que le métal éventuellement contenu dans la charpente de la porphyrine soit le manganèse.

3. Composés complexes selon la revendication 2, caractérisés en ce que V représente un groupe -(CH₂)₂NH-; -CH₂-O-C₆H₄-CH₂-; -CH₂-CH(OH)-CH₂-O-C₆H₄-CH₂-; -C(=NH)-O-C₆H₄- CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂-; -CH₂-CO-NH-(CH₂)₃-O-CH₂-; -CH₂-CO-NH-NH-; -CH₂-CO-NH- -(CH₂)₂-; -CH₂-CO-NH(CH₂)₁₀-; -CH₂-CO-NH-(CH₂)₂-S-; -(CH₂)₄-NH-CO-(CH₂)₈-; -CH₂-CO-NH-(CH₂)₃-NH-; -(CH₂)₃-NH-; -(CH₂)-NH-C(=S)-NH-C₆H₄-CH₂-; -(CH₂)₂-NH-CO-CH₂-(OCH₂CH₂)₄₃-OCH₂-.

4. Utilisation d'au moins un composé complexe de la porphyrine selon la revendication 2 pour la préparation d'agents pour le diagnostic par la RMN.

5. Préparation de composés complexes de la méso-tétraphénylporphyrine constitué d'un ligand de formule générale I et d'au moins un ion paramagnétique d'un élément ayant un nombre atomique de 21 à 29, 42, 44 ou de 57 à 70 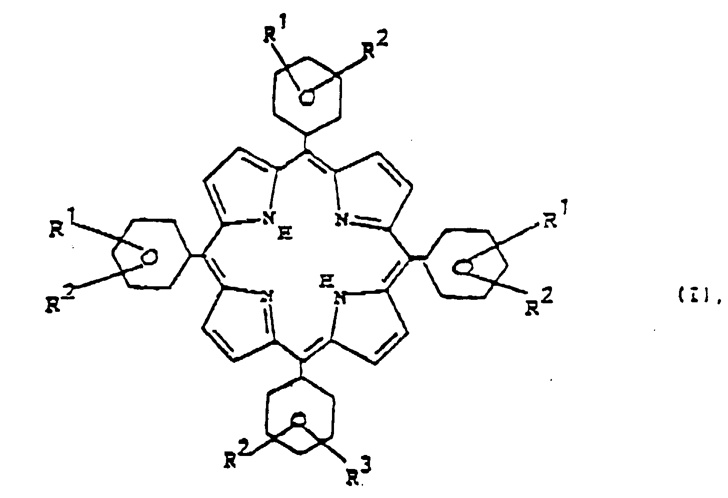 dans laquelle
R¹ représente un radical CO-A, W ou NH-W, avec A étant un groupe (NH)ₓ-[Q-(NH)_{y}]_{w}-W
x et y valent 0, 1 ou 2
w vaut 0 ou 1
Q représente un groupe alkylène en C₁-C₂₀,
W représente le groupement V-K, V étant un groupe alkylène en C₁-C₂₀ linéaire, ramifié, saturé ou insaturé, contenant éventuellement un ou des groupe(s) imino, polyéthylénoxy, phénylène, phénylénoxy, phénylénimino, amido, hydrazido, ester, un ou des atome(s) d'oxygène, de soufre et/ou d'azote, éventuellement substitué par un ou des groupe(s) hydroxy, mercapto, imino, époxy, oxo, thioxo et/ou amino ou représente une liaison directe,
K représente un agent complexant de formules générales IA, IB ou IC 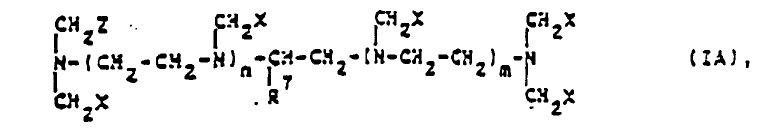 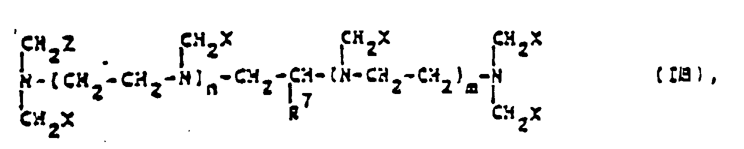 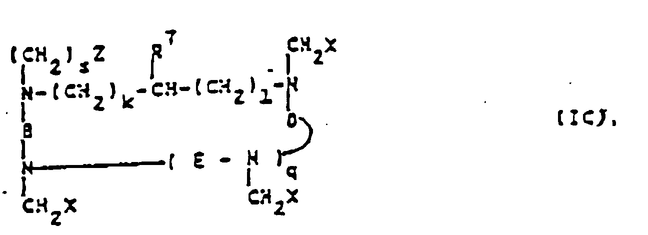 où
n et m valent chacun 0, 1, 2, 3 ou 4, n et m ensemble ne pouvant pas être supérieurs à 4,
k vaut 1, 2, 3, 4 ou 5,
1 vaut 0, 1, 2, 3, 4 ou 5,
q vaut 0, 1 ou 2,
s vaut 0 ou 1,
X représente -COOH
B, D et E, qui sont identiques ou différents, représentent chacun le groupe 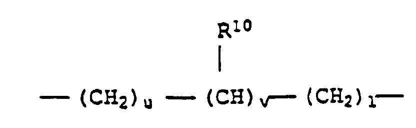 avec
R¹⁰ étant l'hydrogène ou un groupe alkyle en C₁-C₂₀ linéaire, ramifié, saturé ou insaturé, éventuellement contenant un ou des atome(s) d'oxygène, de soufre et/ou d'azote, éventuellement substitué par un ou des groupe(s) hydroxyle et/ou amino,
u vaut 0, 1, 2, 3, 4 ou 5
v vaut 0 ou 1
B, D et E chacun contenant au moins 2 et au maximum 5 atomes de carbone,
Z représente le groupe 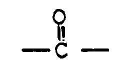 ou le radical X,
R⁷ représente une liaison directe ou un atome d'hydrogène, à la condition que Z ne soit le groupe 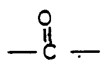 que si R⁷ représente simultanément un atome d'hydrogène, et que Z ne représente le radical X que si R⁷ représente simultanément une liaison directe et s le nombre 1,
R² représente un atome d'hydrogène, de fluor, de chlore, de brome, d'iode ou R⁴, R⁴ étant un alkyle en C₁-C₆ ou un radical benzyle, si on le souhaite, les groupes carboxyles libres encore présents étant partiellement ou entièrement sous la forme d'ester et/ou d'amide ou de sel d'une base organique et/ou inorganique, d'amino-acide ou d'amide d'amino-acide, éventuellement comportant des additifs usuels en galénique, à la condition que le métal éventuellement contenu dans la charpente de la porphyrine soit le manganèse, caractérisé en ce qu'on fait réagir des porphyrines de formule générale II 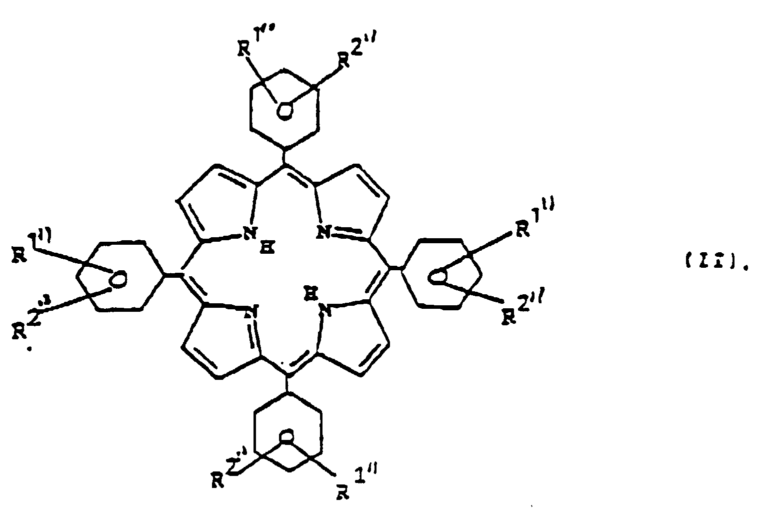
dans laquelle
R^{1"}, représente R^{1'} ou un substituant que l'on peut transformer en R^{1'},
R^{2"} représente un atome d'hydrogène, de fluor, de chlore, de brome, d'iode ou R⁴
R^{1'} et R^{2'} ayant la signification de R¹ et R², toutefois, le substituant W représentant un atome d'hydrogène, éventuellement en transformant de façon connue en soi en porphyrines de formule générale II', dans laquelle R^{1"}, R^{2"} représentent R^{2'}, et ensuite
a) on substitue les groupes NE pyrroliques par l'atome de manganèse, on élimine les groupes protecteurs éventuellement présents et ensuite, on introduit le radical V-K, puis on fait réagir avec au moins un oxyde métallique ou sel métallique d'un élément ayant un nombre atomique de 21 à 29, 42, 44 ou de 57 à 70, ou
b) on introduit le substituant V-K, on fait réagir avec un oxyde métallique ou un sel métallique d'un élément ayant un nombre atomique de 21 à 29, 42, 44 ou de 57 à 70 et ensuite, si on le souhaite, on substitue les groupes NH pyrroliques par un atome de manganèse, ou
c) on introduit le substituant V-K, on substitue les groupes NH pyrroliques par un atome de manganèse et ensuite, on fait réagir avec au moins un oxyde métallique ou sel métallique d'un élément ayant un nombre atomique de 21 à 29, 42, 44, de 57 à 70,
et ensuite, éventuellement on substitue dans les composés complexes obtenus selon a), b) ou c) les atomes d'hydrogène acide encore présents par des cations de bases organiques ou inorganiques, des amino-acides ou des amides d'amino-acides, respectivement on transforme les groupes acides correspondants entièrement ou partiellement en esters et/ou amides.

6. Procédé pour la préparation des agents pharmaceutiques selon la revendication 1, caractérisé en ce qu'on met en forme appropriée pour l'application entérale ou parentérale les composés complexe de porphyrine dissous ou suspendus dans l'eau ou dans une solution physiologique, éventuellement avec des additifs usuels en galénique.
